(19) **Europäisches Patentamt / European Patent Office / Office européen des brevets**

(11) **EP 3 161 479 B1**

(12) **EUROPEAN PATENT SPECIFICATION**

(45) Date of publication and mention of the grant of the patent:
**04.09.2019 Bulletin 2019/36**

(21) Application number: **14742104.4**

(22) Date of filing: **30.06.2014**

(51) Int Cl.:
**G01N 33/50** (2006.01)    **A61K 38/20** (2006.01)

(86) International application number:
**PCT/US2014/044854**

(87) International publication number:
**WO 2016/003409 (07.01.2016 Gazette 2016/01)**

(54) **METHODS FOR ASSESSING THE EFFICACY OF ANTI-INFLAMMATORY CYTOKINE COMPOSITIONS**

VERFAHREN ZUR BEURTEILUNG DER WIRKSAMKEIT VON ENTZÜNDUNGSHEMMENDEN ZYTOKINZUSAMMENSETZUNGEN

PROCÉDÉS POUR ÉVALUER L'EFFICACITÉ DE COMPOSITIONS DE CYTOKINES ANTI-INFLAMMATOIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.05.2017 Bulletin 2017/18**

(73) Proprietor: **Biomet Biologics, LLC Warsaw, Indiana 46582 (US)**

(72) Inventors:
• **TOLER, Krista**
  **Pierceton, Indiana 46562 (US)**
• **KING, William**
  **Warsaw, Indiana 46582 (US)**
• **WOODELL-MAY, Jennifer E.**
  **Warsaw, Indiana 46582 (US)**

(74) Representative: **Handley, Matthew Edward et al Venner Shipley LLP 200 Aldersgate London EC1A 4HD (GB)**

(56) References cited:
WO-A2-2012/030593    US-A1- 2003 138 910
US-A1- 2009 220 482    US-A1- 2010 055 087

• JENNIFER WOODELL-MAY ET AL: "Autologous protein solution inhibits MMP-13 production by IL-1[beta] and TNF[alpha]-stimulated human articular chondrocytes", JOURNAL OF ORTHOPAEDIC RESEARCH, vol. 29, no. 9, 1 September 2011 (2011-09-01), pages 1320-1326, XP055013094, ISSN: 0736-0266, DOI: 10.1002/jor.21384
• KRISTA O'SHAUGHNESSEY ET AL: "Autologous protein solution prepared from the blood of osteoarthritic patients contains an enhanced profile of anti-inflammatory cytokines and anabolic growth factors", JOURNAL OF ORTHOPAEDIC RESEARCH, 1 July 2014 (2014-07-01), pages n/a-n/a, XP055128251, ISSN: 0736-0266, DOI: 10.1002/jor.22671

**Description**

BACKGROUND

The present technology relates to methods for assessing the efficacy of compositions comprising anti-inflammatory cytokines derived from biological materials. Such methods include methods for determining whether a processed biological material is suitable for administering to a subject to treat inflammation mediated by inflammatory cytokines.

Interleukin-1 (IL-1) includes a family of cytokines that can stimulate lymphocytes and macrophages, activate phagocytes, increase prostaglandin production, contribute to degeneration of bone joints, increase bone marrow cell proliferation, and are involved in many chronic inflammatory conditions. IL-1 can be generated by macrophages, monocytes, and dendritic cells, and can be part of the inflammatory response against infection.

The mode of action of IL-1 can be mediated by interleukin-1 receptor antagonist protein (IL-1ra; also known as "IRAP"). IL-1ra binds to the same receptor on the cell surface as IL-1, and thus prevents IL-1 from sending a signal to that cell. IL-1ra is secreted from white blood cells, including monocytes, macrophages, neutrophils, polymorphonuclear cells (PMNs), and other cells, and can modulate a variety of IL-1 related immune and inflammatory responses, as described by Arend WP, Malyak M, Guthridge CJ, Gabay C (1998) "Interleukin-1 receptor antagonist: role in biology" Annu. Rev. Immunol. 16: 27-55. Production of IL-1ra is stimulated by several substances including adherent immunoglobulin G (IgG), other cytokines, and bacterial or viral components. IL-1ra is an important natural anti-inflammatory protein in arthritis, colitis, and granulomatous pulmonary disease.

IL-1ra can be used in the treatment of rheumatoid arthritis, an autoimmune disease in which IL-1 plays a key role, reducing inflammation and cartilage degradation associated with the disease. For example, Kineret™ (anakinra) is a recombinant, non-glycosylated 0 of IL-1ra (Amgen Manufacturing, Ltd., Thousand Oaks, California). Various recombinant interleukin-1 inhibitors and methods of treatment are described in U.S. Patent No. 6,599,873, Sommer et al., issued July 29, 2003; U.S. Patent No. 5,075,222, Hannum et al., issued December 24, 1991; and U.S. Application Publication No. 2005/0197293, Mellis et al., published September 8, 2005. In addition, methods for producing IL-Ira from body fluids, including the use of autologous fluids, are described in U.S. Patent No. 6,623,472, Reincke et al., issued September 23, 2003; U.S. Patent No. 6,713,246, Reinecke et al., issued March 30, 2004; and U.S. Patent No. 6,759,188, Reinecke et al., issued July 6,2004.

Methods for processing biological materials to generate solutions with elevated levels of anti- inflammatory IL-1ra are known in the art. For example, Woodell-May et al. (Journal of Orthopaedic Research vol. 29, No. 9, 1 September 2011, pages 1320-26) describes a Protein Solution produced from platelet-rich plasma, and WO 2012/030593 describes compositions comprising IL-1ra. However, prior art methods may result in elevated levels of inflammatory IL- 1. If the level of IL- 1 in a processed biological material increased more than the level of IL-1ra, then the solution may not be suitable for administration.

There is currently a significant interest in developing devices and methods for processing biological materials to make anti-inflammatory products. However, some devices and methods have been demonstrated to produce outputs with poor ratios of anti-inflammatory proteins to inflammatory proteins due to their generation of inflammatory cytokines in excess of their generation of anti-inflammatory cytokines. Therefore, there is a need to assess the efficacy of products made by such methods and devices so as to attain good clinical outcomes, and to improve and develop devices and methods having increased ratios of anti-inflammatory cytokines to inflammatory cytokines relative to corresponding ratios in the biological material from which the products are made. Also, measurements that describe the efficacy of a processed biological material's anti-inflammatory properties may help medical professionals manage the treatment of anti-inflammatory disorders.

**SUMMARY**

The present invention is defined in the independent claims, to which reference should now be made. Advantageous embodiments are set out in the sub claims. The present technology relates to methods for generating anti-inflammatory compositions. Such methods may comprise:

    a. obtaining a biological material from a subject;
    b. determining input concentrations of an anti-inflammatory cytokine and of an inflammatory cytokine in the biological material, wherein the anti-inflammatory cytokine inhibits the activity of the inflammatory cytokine;
    c. processing the biological material in a device for generating an anti-inflammatory composition to form an anti-inflammatory composition;
    d. determining output concentrations of the anti-inflammatory cytokine and the inflammatory cytokine in the anti-inflammatory composition; and
    e. calculating an Efficacy Ratio.

If the Efficacy Ratio is acceptable, such being 1 or greater, the composition may be prepared for administration to a subject (which may be the subject from whom the biological material is obtained), such as for use used in the treatment of a disorder associated with inflammatory cytokines. Alternatively, if the Efficacy Ratio is unacceptable, such as being less than 1, the composition may be discarded or processed further to increase its Efficacy Ratio.

The present technology also provides methods for treating an inflammatory disorder in a patient. A method for generating an anti-inflammatory composition comprising:

a. obtaining a biological material comprising a first inflammatory cytokine and a first anti-inflammatory cytokine from a human or other animal subject;

b. determining the input concentration of the first anti-inflammatory cytokine ([first anti-inflammatory cytokine] input), and the input concentration of the first inflammatory cytokine ([first inflammatory cytokine] input), in the biological material;

c. processing the biological material to produce an anti-inflammatory composition having an increased concentration of the first anti-inflammatory cytokine;

d. determining output concentration of the first anti-inflammatory cytokine ([first anti-inflammatory cytokine] output) and the output concentration of the first inflammatory cytokine ([first inflammatory cytokine] output), in the anti-inflammatory composition;

e. calculating a first Efficacy Ratio pursuant to the formula:

$$\text{first Efficacy Ratio} = \frac{\left(\frac{[\text{first anti} - \text{inflammatory cytokine}]}{[\text{first inflammatory cytokine}]}\right)\text{Output}}{\left(\frac{[\text{first anti} - \text{inflammatory cytokine}]}{[\text{first inflammatory cytokine}]}\right)\text{Input}}$$

and;

f. preparing the anti-inflammatory composition for administration to the subject if the Efficacy Ratio is acceptable (e.g., 1 or greater), or discarding the anti-inflammatory composition, or repeating steps (a) through (e), if the first Efficacy Ratio is not acceptable (e.g., if less than 1).

In various methods the biological material is whole blood, a blood fraction (such as platelet rich plasma), adipose tissue, adipocytes, bone marrow aspirate, concentrated bone marrow aspirate, synovial fluid, urine, or mixtures thereof. The anti-inflammatory cytokine may comprise a cytokine selected from the group consisting of interleukin-1 receptor antagonist (IL-1ra), soluble IL-1 receptor II (sIL-1RII), interleukin-4 (IL-4), interleukin-10 (IL-10), interleukin-13 (IL-13), soluble tumor necrosis factor receptor I (sTNF-RI), soluble tumor necrosis receptor II (sTNF-RII), and mixtures thereof.

DRAWINGS

The drawings described herein are for illustrative purposes only of selected embodiments and not all possible implementations, and are not intended to limit the scope of the present disclosure.

Fig. 1 is a block diagram illustrating a method for producing an anti-inflammatory cytokine composition;
Fig. 2 is a diagram of a fractionation device;
Figs. 3A and 3B show a device for activating a sample to generate anti-inflammatory cytokines, before (Fig. 3A) and after (Fig. 3B) centrifugation;
Fig. 4 is a diagram of a device for generating a blood clot;
Fig. 5 is a diagram of a single device capable of generating an anti-inflammatory cytokine composition;
Fig. 6 is a block diagram illustrating a method for generating an anti-inflammatory composition; and
Fig. 7 is a block diagram illustrating a method for treating an inflammatory disorder in a patient.

DETAILED DESCRIPTION

The following description of technology is merely exemplary in nature of the composition, manufacture and use of one or more inventions, and is not intended to limit the scope, application, or uses of any specific invention claimed in this application or in such other applications as may be filed claiming priority to this application, or patents issuing therefrom.

The present technology provides to methods for assessing the efficacy of anti-inflammatory compositions made from biological materials. Such methods may be used in a variety of ways in designing and producing devices and compositions and in clinical practice, so as to measure, control, improve, assess quality, and otherwise manage the production of biologically-derived anti-inflammatory compositions and their use in treating disorders associated with inflammatory cytokines. For example, such methods may be used to assess devices used in making such anti-inflammatory compositions, methods for generating such compositions, and in methods for determining whether an anti-inflammatory composition is suitable for treating an inflammatory disorder.

Many inflammatory disorders are associated with increased levels of inflammatory cytokines at a site of inflammation. Such inflammatory disorders include rheumatoid arthritis, osteoarthritis, osteolytis, tendonitis, synovitis, peripheral vascular disease, and inflammatory respiratory diseases (such as chronic obstructive pulmonary disease, fibrosis, emphysema, acute respiratory distress syndrome, and pneumonia). Inflammatory cytokines commonly associated inflammatory disorders include interleukin-la (IL-1$\alpha$), interleukin-1$\beta$ (IL-1$\beta$), and tumor necrosis factor $\alpha$ (TNF$\alpha$). Collectively, IL-1$\alpha$ and IL-1$\beta$ are referred to as "IL-1."

Various biological tissues can express anti-inflammatory cytokines that mediate the inflammatory activity of inflammatory cytokines. For example, the mode of action of IL-1 can be mediated by interleukin-1 receptor antagonist (IL-1ra). IL-1ra binds to the same receptor on the cell surface as IL-1, and thus prevents IL-1 from sending a signal to that cell. IL-1ra is secreted from white blood cells, including monocytes, macrophages, neutrophils, polymorphonuclear cells (PMNs), and other cells, and can modulate a variety of IL-1 related immune and inflammatory responses, as described by Arend WP, Malyak M, Guthridge CJ, Gabay C (1998) "Interleukin-1 receptor antagonist: role in biology" Annu. Rev. Immunol. 16: 27-55. Production of IL-1ra is stimulated by several substances including adherent immunoglobulin G (IgG), other cytokines, and bacterial or viral components. Additionally, the mode of action of IL-1 can be mediated by soluble interleukin-1 receptor II (sIL-1RII). sIL-1RII is a soluble receptor that mediates the activity of IL-1$\alpha$ and IL-1$\beta$ by binding to IL-1$\alpha$ and IL-1$\beta$, which prevents IL-1$\alpha$ and IL-1$\beta$ from activating IL-1 receptors (IL-IRs). Likewise, the mode of action of TNF-$\alpha$ can be mediated by soluble tumor necrosis factor receptor I (sTNF-RI) and soluble tumor necrosis factor receptor II (sTNF-RII), which prevent TNF-$\alpha$ from binding to membrane bound TNF-RI and/or TNF-RII. Additional anti-inflammatory cytokines include interleukin-4 (IL-4), interleukin-10 (IL-10), and IL-13.

Inflammatory disorders can be treated with an allogeneic or autologous anti-inflammatory composition that is generated in a device from a biological material. However, the ratio of anti-inflammatory cytokines to inflammatory cytokines in the anti-inflammatory compositions generated by the devices should be greater than the same ratio in the biological material from which the anti-inflammatory composition was generated. Accordingly, the present technology provides an Efficacy Ratio, as described further below, to aid the measurement, production, quality control and other management of the design, production, and use of biologically-derived anti-inflammatory compositions in treating disorders associated with inflammatory cytokines.

Protein Compositions

The present technology provides anti-inflammatory compositions (e.g., "Protein Solutions") comprising proteins dissolved, suspended or otherwise carried for delivery to a mammalian subject in a physiologically-acceptable medium. In various embodiments, such compositions comprise proteins (e.g., cytokines) that are native to whole blood in normal mammal subjects. Such compositions may also contain viable cells, including platelets, white blood cells, and combinations thereof. In some embodiments, the medium may be a concentrated plasma solution.

In various embodiments, the Protein Solution comprises at least two proteins selected from the group consisting of IL-1ra (interleukin-1 receptor antagonist), sTNF-RI, sTNF-RII (soluble tumor necrosis factor-receptor 2), interleukin-4 (IL-4), interleukin-10 (IL-10), interleukin-13 (IL-13), and sIL-1RII (soluble interleukin one receptor II), wherein the concentration of each protein in the composition is greater than the concentration of the protein in normal blood. In various embodiments, the protein compositions have an efficacy ratio equal to or greater than 1, as set forth below. For the sake of clarity, the Protein Solution may contain three or more of the proteins from the recited group. While the concentration of every such protein in the composition may be greater than its respective concentrations in normal blood, it is not necessary that the concentration of more than two of the proteins be greater than their respective concentrations in normal blood.

In various embodiments, the platelet-rich protein solution comprises the following components.

Table 1. Protein Solution Exemplary Protein Components.

| Component | Composition Concentration | Normal Whole Blood Concentration |
|---|---|---|
| plasma proteins (total) | about 80 mg/ml or greater<br>about 100 mg/ml or greater<br>about 200 mg/ml or greater<br>about 250 mg/ml or greater | about 67 mg/ml |

(continued)

| Component | Composition Concentration | Normal Whole Blood Concentration |
|---|---|---|
| albumin | about 60 mg/ml or greater<br>about 100 mg/ml of greater | about 56 mg/ml |
| fibrinogen | about 3.2 mg/ml or greater<br>about 4 mg/ml or greater | about 2.9 mg/ml |
| IL-1ra | about 10,000 pg/ml or greater<br>about 25,000 pg/ml or greater<br>about 30,000 pg/ml or greater<br>from about 25,000 to about 110,000 pg/ml<br>from about 25,000 to about 40,000 pg/ml | about 4200 pg/ml |
| IL-4 | about 5 pg/ml or greater<br>about 10 pg/ml or greater<br>about 20 pg/ml or greater | about 0 pg/ml |
| IL-10 | about 2 pg/ml or greater<br>about 5 pg/ml or greater | about 1 pg/ml |
| sTNF-RI | about 1,200 pg/ml or greater<br>about 1,800 pg/ml or greater<br>about 3,000 pg/ml or greater | about 630 pg/ml |
| sTNF-RII | about 3,000 pg/ml or greater<br>about 5,000 pg/ml or greater<br>about 7,000 pg/ml or greater<br>about 9,000 pg/ml or greater | about 1200 pg/ml |
| sIL-1RII | about 15,000 pg/ml or greater<br>about 20,000 pg/ml or greater<br>about 25,000 pg/ml or greater | about 11,800 pg/ml |
| Growth factors | | |
| EGF | about 800 pg/ml or greater<br>about 1,000 pg/ml or greater<br>about 1,200 pg/ml or greater | about 250 pg/ml |
| HGF | about 1,000 pg/ml or greater<br>about 2,500 pg/ml or greater<br>about 2,800 pg/ml or greater<br>about 3,000 pg/ml or greater | about 500 pg/ml |
| PDGF- AB | about 35,000 pg/ml or greater<br>about 50,000 pg/ml or greater<br>about 70,000 pg/ml or greater | about 6,000 pg/ml |
| PDGF- BB | about 10,000 pg/ml or greater<br>about 15,000 pg/ml or greater<br>about 20,000 pg/ml or greater | about 1,500 pg/ml |
| TGF-$\beta$1 | about 100,000 pg/ml or greater<br>about 150,000 pg/ml or greater<br>about 190,000 pg/ml or greater | about 10,000 pg/ml |
| IGF-1 | about 130,000 pg/ml or greater<br>about 150,000 pg/ml or greater<br>about 160,000 pg/ml or greater | about 70,000 pg/ml |

(continued)

| Growth factors | | |
|---|---|---|
| VEGF | about 500 pg/ml or greater<br>about 600 pg/ml or greater<br>about 800 pg/ml or greater | about 150 pg/ml |

Protein concentrations can be measured using the methods set forth in Example 4.

The composition further preferably comprises viable white blood cells, lysed white blood cells, or both. In a preferred composition, the Protein Solution comprises monocytes, granulocytes, and platelets. In various embodiments, a Protein Solution comprises the following components.

Table 2. Protein Solution Exemplary Cellular Components.

| Component | Composition Concentration | Normal Whole Blood Concentration |
|---|---|---|
| white blood cells | at least about 15 k/$\mu$l<br>at least about 30 k/$\mu$l<br>from about 30 to about 60 k/$\mu$l<br>from about 40 to about 50 k/$\mu$l | 6.5 k/$\mu$l |
| red blood cells | less than about 3 M/$\mu$l<br>less than about 2 M/$\mu$l<br>less than about 2.5 M/$\mu$l | 4.5 M/$\mu$l |
| platelets | at least about 400 k/$\mu$l<br>at least about 800 k/$\mu$l<br>at least about 1,000 k/$\mu$l | 240 k/$\mu$l |
| neutrophils | at least about 5 k/$\mu$l<br>at least about 10 k/$\mu$l<br>at least about 12 k/$\mu$l | 3.7 k/$\mu$l |
| monocytes | at least about 1 k/$\mu$l<br>at least about 2 k/$\mu$l<br>at least about 3 k/$\mu$l | 0.5 k/$\mu$l |
| lymphocytes | at least about 5 k/$\mu$l<br>at least about 10 k/$\mu$l<br>at least about 20 k/$\mu$l | 2 k/$\mu$l |
| eosinophiles | at least about 0.15 k/$\mu$l<br>at least about 0.18 k/$\mu$l | 0.1 k/$\mu$l |
| basophils | at least about 0.2 k/$\mu$l<br>at least about 0.4 k/$\mu$l<br>at least about 0.6 k/$\mu$l | 0.1 k/$\mu$l |

It will be understood that this concentration is species specific. Further, it is understood that concentrations may vary among individual subjects. Thus, in methods comprising production of a Protein Solution from the blood or other tissue containing cytokine-producing cells, the concentration of proteins and cells in the Protein Solution may vary from those recited above; the values recited above are mean values for concentrations as may be seen in a population of subjects.

In various embodiments, the concentration of one or more of the proteins or other components in the Protein Solution is greater than the concentration of the component in normal blood. (Compositions with such higher concentrations of components are said to be "rich" in such components.) As referred to herein, the concentration of a component in "normal" blood or other tissue is the concentration found in the general population of mammalian subjects from which the tissue is obtained, e.g., in normal whole blood. In methods wherein the anti-inflammatory cytokine composition is derived from tissue from a specific subject, the "normal" concentration of a protein or cell may be the concentration in the blood of that individual before processing is performed to derive the protein or cell.

Thus, in various embodiments, the concentration of one or more components of the Protein Solution is greater than about 1.5 times, about 2 times, or about 3 times, greater than the concentration of the component in normal blood. For example, components may have greater concentrations in the compositions, relative to normal (whole) blood, as follows:

- IL-1ra, at a concentration that is at least about 2.5, or at least about 3 or at least about 5, times greater;
- IL-4, at a concentration that is at least about 2.5, or at least about 3 or at least about 5, times greater;
- IL-10, at a concentration that is at least about 1.5, or at least about 2 or at least about 2.5, times greater;
- sTNF-RI, at a concentration that is at least about 2, or at least about 2.5 or at least about 3, times greater;
- sTNF-RII, at a concentration that is at least about 2, or at least about 2.5 or at least about 3, times greater;
- sIL-1RII, at a concentration that is at least about 1.5, or at least about 1.8 or at least about 2, times greater;
- EGF, at a concentration that is at least about 2, or at least about 3 or at least about 5, times greater;
- HGF, at a concentration that is at least about 2, or at least about 3 or at least about 4, times greater;
- PDGF-AB, at a concentration that is at least about 2, or at least about 3 or at least about 5, times greater;
- PDGF-BB, at a concentration that is at least about 2, or at least about 3 or at least about 5, times greater;
- TGF-$\beta$1, at a concentration that is at least about 3, or at least about 4 or at least about 6, times greater;
- IGF-1, at a concentration that is at least about 1.2, or at least about 1.4 or at least about 1.5, times greater;
- VEGF, at a concentration that is at least about 2, or at least about 2.5 or at least about 3, times greater;
- white blood cells, at a concentration that is at least about 2, or at least about 3 or at least about 4, times greater;
- platelets, at a concentration that is at least about 2, or at least about 3 or at least 4, times greater;
- neutrophils, at a concentration that is at least 1.5, or at least 2 or at least 3, times greater;
- monocytes, at a concentration that is at least 3, or at least 4 or at least 6, times greater;
- lymphocytes, at a concentration that is at least 5, or at least 8 or at least 10, times greater; and
- basophils, at a concentration that is at least 2, or at least 4 or at least 6, times greater.

Also, the concentration of erythrocytes in the Protein Solution is preferably at least half, or at least a third, of the concentration of erythrocytes in normal blood.

For example, a Protein Solution may comprise:

(a) at least about 10,000 pg/ml IL1-ra;
(b) at least about 1,200 pg/ml sTNF-RI; and
(c) a protein selected from the group consisting of sTNF-RII, IGF-I, EGF, HGF, PDGF-AB, PDGF-BB, VEGF, TGF-$\beta$1, and sIL-1RII, and mixtures thereof, wherein the protein has a concentration higher than the protein's baseline concentration in normal blood.

In another example, a Protein Solution comprises:

(a) interleukin-1 receptor antagonist (IL-1ra), at a concentration at least 3 times greater than the concentration of IL-1ra in normal blood;
(b) soluble tissue necrosis factor-r1 (sTNF-r1), at a concentration at least 2 times greater than the concentration of IL-1ra in normal blood;
(c) white blood cells at a concentration at least 2 times greater than the concentration of white blood cells in normal blood; and
(d) platelets, at a concentration at least 2 times greater than the concentration of platelets in normal blood.

In some embodiments, the concentration of IL-1ra in the Protein Solution is preferably at least 5,000, or at least 10,000, times greater than the concentration of interleukin-la in the Protein Solution. The ratio of IL-1ra:interleukin-1$\beta$ (IL-1 $\beta$) concentrations is preferably at least 100. In some embodiments, the concentration of IL-1ra in the Protein Solution is preferably at least 1500, or at least 8000, times greater than the concentration of IL-1 $\beta$ in the Protein Solution. The ratio of sIL-1RII:interleukin-1$\beta$ (IL-1 $\beta$) concentrations is preferably greater than 1.

In various embodiments, the Protein Solution comprises one or more components (e.g., platelets) derived from the subject to whom the solution is to be administered in a treatment methods according to this technology. Such components are, accordingly, "autologous." In some embodiments, the Protein Solutions (e.g., Autologous Protein Solutions) consisting essentially of such autologous components. In other embodiments, one or more components of the solution may be obtained from non-autologous sources, such as through recombinant or synthetic methods, or by isolation from allogeneic sources (i.e., from subjects of the same species as the subject to whom the solution is to be administered) or xenogeneic sources (i.e., from animal sources other than the species to whom the solution is to be administered).

Methods of Making Protein Solutions

Protein Solutions may be made by any of a variety of methods, including admixture of individual components and processes wherein one or more components are derived from a source material. In various embodiments, the Protein Solution is made by fractionating a cytokine cell suspension, to produce a protein solution comprising IL1-ra. In other embodiments, the Protein Solution is made with an efficacy ratio within a specific range, as described herein.

Obtaining Protein Solutions by Contacting Cytokine-producing cells with an Extraction Material

In various embodiments, Protein Solutions are made by derivation of one or more components from tissue comprising cytokine-producing cells. As referred to herein, a "cytokine producing tissue" is a tissue obtained from a mammalian subject, comprising cells that are capable of producing cytokines. Such cells include white blood cells, adipose stromal cells, bone marrow stromal cells, and combinations thereof. It is understood that white blood cells include monocytes, lymphocytes, and granulocytes such as neutrophils, eosinophils, and basophils. White blood cells useful in the methods of this technology preferably include monocytes and neutrophils. Cytokine producing tissues among those useful herein include blood, adipose tissue, bone marrow, and fractions thereof, as further discussed below.

Blood useful herein includes whole blood, plasma, platelet-rich plasma, platelet-poor plasma, and blot clots. In a preferred embodiment, methods of the present technology use platelet-rich plasma (PRP), containing white blood cells and platelets, comprising the buffy coat layer created by sedimentation of whole blood. Adipose tissue useful herein includes any fat tissue, including white and brown adipose tissue, which may be derived from subcutaneous, omental/visceral, mammary, gonadal, or other adipose tissue sites. Bone marrow useful herein includes red marrow and yellow marrow. In a preferred embodiment, bone marrow is bone marrow concentrate, obtained from the red marrow of long bones, comprising hematopoietic and mesenchymal stems cells. As discussed above, blood, adipose, and bone marrow tissue useful herein may be from either autologous or allogeneic sources, relative to the subject to be treated according to methods of this technology. Compositions may also be made from combinations of allogeneic and autologous tissues.

In some embodiments, methods comprise fractionating a liquid (a "cytokine cell suspension") comprising cells capable of producing cytokines, such as IL1-ra and sTNF-R1. As discussed above, such cells include white blood cells, adipose stromal cells, bone marrow stromal cells, and combinations thereof. In some embodiments, the cytokine cell suspension is a liquid comprising white blood cells. It should be understood that the cytokine cell suspension comprises cells and an extracellular liquid, regardless of the relative proportions of the cells and liquid. In some embodiments, the suspension may comprise primarily cells, with liquid being present as only a minor component, essentially wetting the cells. In some embodiments, the liquid may comprise two phases, consisting of a phase primarily consisting of liquid and a phase primarily consisting of cells, forming a suspension of cells in the liquid only upon agitation or other mixing.

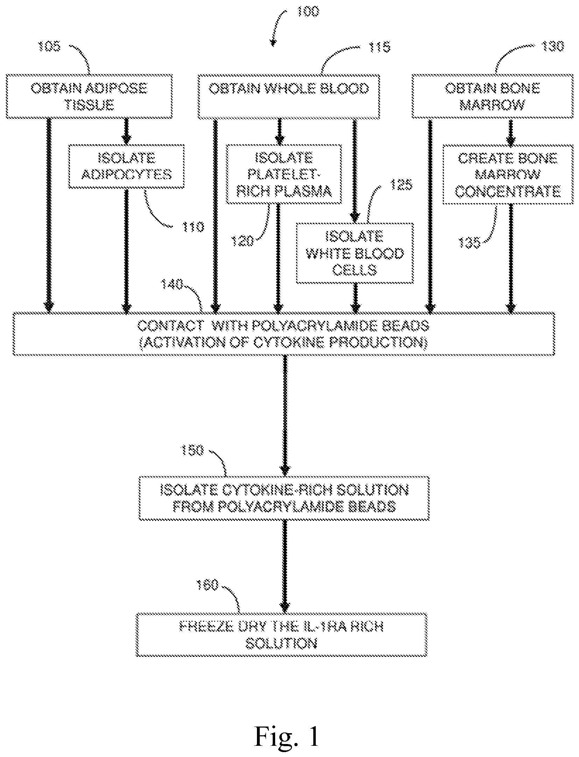

In some embodiments, fractionating a cytokine cell suspension comprises contacting the liquid with a solid extraction material. As exemplified in Fig. 1, such processes comprise:

(a) obtaining a cytokine cell suspension, such as a liquid comprising white blood cells (steps 105, 115 or 135, or combinations thereof);
(b) contacting the tissue with a solid extraction material (step 140); and
(c) isolating a protein-containing liquid from the solid extraction material (step 150).

Obtaining the suspension 105, 115, 135 can comprise any of a variety of methods for creating a liquid containing cells among those known in the art. Such methods include isolation from tissue and culturing. Obtaining may be performed directly in the method, whereby a health care practitioner or other individual performs isolation, processing, culturing or other processes for creating the suspension, in a procedure that includes the contacting and isolating steps. In some embodiments, the processes for creating the suspension are performed contemporaneously with the contacting and isolating steps, as part of a point-of-care procedure, as discussed further herein. Alternatively, obtaining the suspension may be indirect, involving only the acquisition of the suspension for use in the contacting and isolating steps, wherein the processing to create the suspension has previously been performed by another party.

In various embodiments, obtaining comprises isolating a cytokine cell suspension, comprising white blood cells or other cytokine-producing cells, from blood, adipose tissue, bone marrow aspirate or other tissue comprising cytokine-producing cells, as exemplified in Steps 110, 120 and 125 of Fig. 1. Methods may comprise obtaining a cytokine cell suspension from two, three or more tissue sources.

Obtaining a Cytokine cell suspension from Blood

In embodiments comprising the use of blood, the blood may be used directly in contacting the solid extraction material, as exemplified in step 140 of Fig. 1, or may be processed to provide a blood fraction, such as PRP, in a preferred embodiment. Many devices and methods for creating blood fractions are known in the art, using such means as centrifugation and filtering.

In various embodiments, methods of the present technology comprise creating PRP as the cytokine cell suspension, using centrifugation. Such methods generally comprise placing blood in a container a separator operable to separate the blood into two or more fractions, and centrifuging the separator to create a platelet-rich plasma fraction. Such devices may include a tube and a buoy disposed in the tube, wherein the buoy has a density such that the buoy reaches an equilibrium position upon centrifugation of the tissue in the tube, the equilibrium position being between a first fraction and a second fraction comprising cytokine-producing cells, the second fraction having a concentration of cytokine-producing cells greater than the concentration of cytokine-producing cells in the first fraction. Such methods further comprise centrifuging the tube so that the buoy defines an interface between the first fraction and the second fraction comprising cytokine-producing cells. The second fraction is then collected for further use in the methods of this technology.

One such device useful herein is described in U.S. Patent 7,992,725, Leach et al., issued August 9, 2011. Such a device is commercially available as GPS III Platelet Concentrate and Separation System, from Biomet Biologics, LLC (Warsaw, Indiana, USA). The device can be used in a clinical or laboratory environment to isolate fractions from a suspension or multi-component tissue material obtained from a subject, such as blood, bone marrow aspirate, cerebrospinal fluid, adipose tissue, Isolated fractions can include platelets, platelet poor plasma, platelet rich plasma and stromal cells. The isolated fractions can each have equilibrium point or positions within the separation container that are achieved when separation has occurred. For example, a buffy coat (PRP) of whole blood may have an equilibrium position above that of the red blood cells when a sample of whole blood is separated.

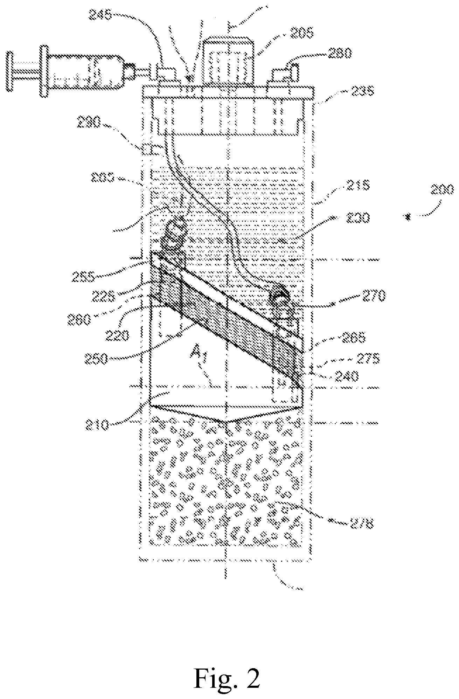

The fractionation device 200 is exemplified in Fig. 2. The fractionation device 200 comprises a buoy 210 and a container wall 215. When the separation container 205 is centrifuged, the buoy perimeter 210a and the container wall 215 have clearance allowing the buoy 210 to move within the separation container 205 and a material to pass between the buoy perimeter 210a and the container wall 215. Alternatively, the buoy 210 could have an opening, such as a centrally or internally located opening or a peripheral channel running the height of the buoy, which would allow a material to move through the buoy.

The buoy 210 is carried in the separation container 205 and has a tuned density that is configured to reach a selected equilibrium position in a suspension. The buoy can have its density tuned in the range from about 1 g/cc to about 1.10 g/cc, such as about 1.06 g/cc. The buoy 210, according to various embodiments, can be formed to include the tuned density and can be formed of one or more materials to achieve the tuned density.

Referring to Fig. 2, a collection area 220 is positioned within the device 200 after a separation procedure has occurred. The collection area 220, defined relative to the buoy 210, is positioned at an equilibrium position of a separated or isolated middle fraction 225 in the container. The equilibrium position of a selected fraction can be defined as its position within the container relative to other fractions in the container of a separated sample or material. The equilibrium position can also be defined relative to the axis X of the buoy 210 or the container 12. The equilibrium position, however, may depend upon the amount of the sample of the amount of a selected fraction within a sample. According to the illustration in Fig. 2, the equilibrium position of the fraction 230 is above or nearer a top 235 of the device 200 than the equilibrium position of the fraction 225. Thus, the buoy 210 can be tuned, such as including a selected density or specific gravity, to position the collection area 220 relative to an equilibrium position of any selected fraction.

In some embodiments, the buoy 210 can comprise a collection port 240. The collection port 240 communicates with access port 245 and communicates with a collection space 220 above buoy upper surface 250 and can be located near the buoy perimeter 210a. In some embodiments, the collection port 240 is not carried on the buoy, but rather the collection port is a withdraw device such as a syringe that is inserted through an access port or top of the device 200.

According to various embodiments, an isolator 255 is coupled to the buoy 210. The combination of the isolator and buoy, according to various embodiments, can also be referred to as a separation assembly member. The isolator 255, for example, provides a means for creating the collection compartment 220 and comprises one or more spacers 260, 265 to position the isolator 255 apart from the buoy 210 to create the collection compartment 220. A withdraw port 270 can be carried on the isolator 255 communicating with the withdraw port 245 and the collection port 240. The spacer 260, 265 can also serve as a conduit 275 between the collection port 50 and a withdraw or withdraw port 245. The withdraw port 245 serves as a structure for withdrawing the isolated or second fraction 310 from the collection compartment 220.

After centrifuging the device 200 containing whole blood, the first fraction or top fraction 230, can be platelet-poor-plasma, the middle fraction 225 can be platelet-rich plasma or platelet concentrate, and a bottom fraction 278 can be red blood cells. Therefore, the fractionation method further comprises withdrawing a desired fraction from the device 200. Various ports 205, 245 and 280 can be provided to allow access to any appropriate compartment of the device 200. The access ports 205, 245, 280 can be any means that allow communication from outside the separation device 200 to the device's interior, such as a Luer lock port, a septum, a valve, or other opening. Additionally, collection vent tube 285 allows removal of a fractionated suspension in the collection area 220 through opening 290 without the need to remove the fraction, such as plasma, above the isolator 255. Although, without a collection vent tube 285, the fraction above the isolator could be removed and the collection area could be vented to the area above the isolator.

A method for using the fractionation device 200 can begin by inputting whole blood via an access port 205. The fractionation device 200 is placed into a centrifuge and spun for a period that is appropriate for fractionating whole blood. An exemplary period can be for about five minutes to about twenty minutes at a rate of about 320 rpm to about 5000 rpm. This speed may produce a selected gravity that may be approximately 7.17 xg to about 1750 xg (times greater than the normal force of gravity).

Other devices that may be used to isolate platelet-rich plasma described, for example, in U.S. Patent 5,585,007, Antanavich, issued December 17, 1996; U.S. Patent 6,398,972, Blasetti et al., issued June 4, 2002; U.S. Patent 6,649,072, Brandt et al., issued November 18, 2003; U.S. Patent 6,790,371, Dolocek, issued September 14, 2004; U.S. Patent 7,011,852, Sukavaneshvar et al., issued March 14, 2006; U.S. Patent 7,179,391, Leach et al., issued February 20, 2007; U.S. Patent 7,374,678, Leach et al., issued May 20, 2008; U.S. Patent 7,223,346, Dorian et al., issued May 29, 2007; and U.S. Patent 7,708,152, Dorian et al., issued May 4, 2010.

In addition to the GPS® Platelet Concentrate and Separation Systems, a variety of other commercially available devices may be used to isolate platelet-rich plasma, including the Magellan™ Autologous Platelet Separator System, commercially available from Medtronic, Inc. (Minneapolis, Minnesota, USA); SmartPReP™, commercially available from Harvest Technologies Corporation (Plymouth, Massachusetts, USA); the AutoloGel™ Process, commercially available from Cytomedix, Inc. (Rockville, Maryland, USA); the GenesisCS System, commercially available from EmCyte Corporation (Fort Myers, Florida, USA); the PCCS System, commercially available from Biomet 3i, Inc. (Palm Beach Gardens, Florida, USA); and the Arthrex ACP™ Double Syringe System, commercially available from Arthrex, Inc. (Naples, Florida, USA).

Referring again to Fig. 1, blood drawn from the patient may be mixed with an anticoagulant in one or more of Steps 115, 120, 125, and 130, so as to facilitate processing. Suitable anticoagulants include heparin, citrate phosphate dextrose (CPD), ethylenediaminetetraacetic acid (EDTA), anticoagulant citrate dextrose solution (ACD), and mixtures thereof. For example, the anticoagulant may be placed in the syringe used for drawing blood from the subject, or may be mixed with the blood after it is drawn.

A cytokine cell suspension may be prepared by admixing cells with a suitable liquid, as shown in step 125, using methods known in the art. For example, white blood cells may be isolated from whole blood by lysing red blood cells or by centrifugation of whole blood utilizing a density gradient where the white blood cells sediment to the bottom of a centrifuge tube. An example of density centrifugation includes the Ficoll-Paque™ Plus (GE Healthcare Bio-Sciences, Piscataway, New Jersey, USA). In some cases, a density gradient may be used to further separate mononuclear and polymorphonuclear cells. Cytokine-producing cells may also be prepared from whole blood using filtration; an example includes the Acelere™ MNC Harvest System (Pall Life Sciences, Ann Arbor, Michigan, USA). Cytokine-producing cells can also be obtained from bone marrow. The cytokine-producing cells may be then suspended in a suitable medium, such as plasma, so as to maintain their viability.

Other methods may be used to create platelet-rich plasma or other cytokine cell suspension. For example, whole blood can be centrifuged without using a buoy system, whole blood may be centrifuged in multiple stages, continuous-flow centrifugation can be used, and filtration can also be used. In addition, a blood component including platelet-rich plasma can be produced by separating plasma from red blood cells using a slow speed centrifugation step to prevent pelleting of the platelets. In other embodiments, the buffy coat fraction formed from centrifuged blood can be separated from remaining plasma and re-suspended to form platelet-rich plasma.

Obtaining a Cytokine Cell Suspension from Adipose Tissue

In embodiments comprising the use of adipose tissue, the adipose tissue may be used directly in contacting the solid extraction material, as exemplified in step 140 of Fig. 1, or the adipose tissue may be processed to provide isolated adipocytes in step 110. Cell fractions comprising adipose-derived stem cells are also useful in this method. In some embodiments, adipose tissue is derived from human subcutaneous fat isolated by suction assisted lipectomy or liposuction. Stromal cells may be isolated from the adipose tissue and/or tissue portions using any suitable method, including methods known in the art such as mechanical and breakdown centrifugation. Stromal cells can also be isolated using enzymatic digestion. For example, stromal cells can be isolated from lipoaspirate, treated by sonication and/or enzymatic digestion, and enriched by centrifugation. Stromal cells isolated from adipose tissue may be washed and pelleted.

For example, adipose tissue can be collected by suction-assisted tumescent liposuction inside a specialized collection container attached to suction hoses and to a liposuction cannula. The collection container can have a gauze-type grid filter that allows the tumescent fluid to pass through and retains the solid adipose tissue. After collecting the adipose tissue, the collection container is removed from the suction device and reattached to a centrifugation device.

The filter unit may further contain a filter having approximately a 100 micrometer pore size. Once the collection container containing the adipose tissue is attached to the centrifugation device, the tissue is sonicated. After sonication, the entire apparatus is inserted into a centrifuge bucket and centrifuged at, for example, 300×g for 5 minutes. After centrifugation, the collection container together with the filter unit is detached and can be discarded. The pellet containing the stromal cells can then be re-suspended in biocompatible solutions, such as plasma, plasma concentrate and platelet-rich plasma.

Various methods and devices for isolating and/or fractionating adipose tissue and adipocytes include those as described by U.S. Patent 7,374,678, Leach, issued May 20, 2008; U.S. Patent 7,179,391 to Leach et al., issued February 20, 2007; U.S. Patent 7,992,725, Leach et al., issued August 9, 2011; U.S. Patent 7,806,276, Leach et al., issued October 5, 2010; and U.S. Patent 8,048,297, Leach et al., issued November 1, 2011. A device, such as the GPS™ Platelet Concentrate System, commercially available from Biomet Biologics, LLC (Warsaw, Indiana, USA), may be used to isolate adipocytes.

Obtaining a Cytokine cell suspension from Bone Marrow

In embodiments comprising the use of bone marrow, the marrow may be used directly in contacting the solid extraction material, as exemplified in step 140 of Fig. 1, or may be processed to provide a bone marrow concentrate, as in step 135. Many devices and methods for obtaining and concentrating bone marrow are known in the art.

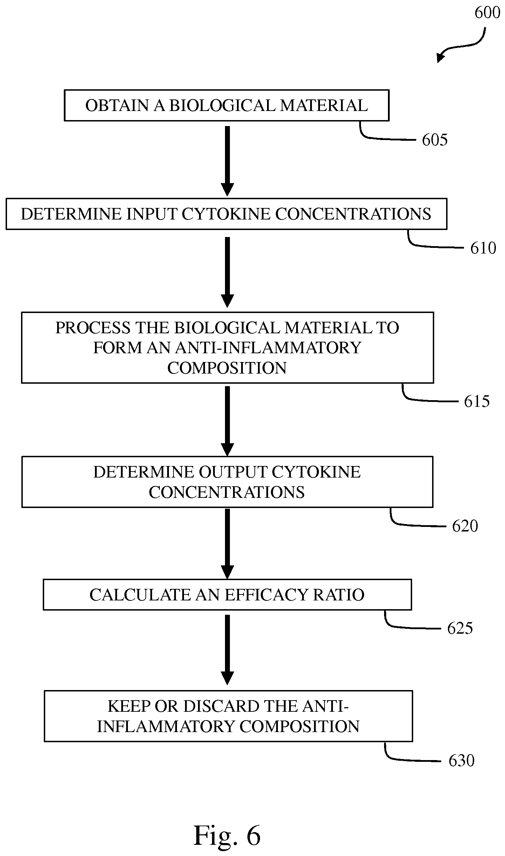

An exemplary process for isolating and creating a bone marrow concentrate (cBMA) is diagrammed in Fig. 6. Generally, the method 600 may start in step 605 with obtaining a bone marrow aspirate volume. The bone marrow aspirate (BMA) may be obtained in any selected or generally known manner. For example, a selected region of bone, such as a portion near an operative procedure, may be used to obtain the bone marrow aspirate. Generally, an accessing device, such as a syringe and needle, may be used to access an intramedullary area of a selected bone. A small volume of the selected portion may be drawn from a plurality of locations to obtain an appropriate volume of BMA or selected fraction of the BMA.

Once a selected volume of the BMA is obtained in step 605, it may be separated and concentrated using a gravimetric separator. Separators among those useful herein are operable to separate a multi-component fluid that generally includes various components or constituents of varying densities that are commingled or mixed together, including those described above for separation of fractions from blood and adipose tissue. The separator may include a buoy that is of a selected density relative to BMA. Such separators include those described above for use in concentrating and isolating fractions from blood and adipose tissue, including those described in U.S. Patent 7,374,678, Leach, issued May 20, 2008; U.S. Patent 7,179,391 to Leach et al., issued February 20, 2007; U.S. Patent 7,992,725, Leach et al., issued August 9, 2011; U.S. Patent 7,806,276, Leach et al., issued October 5, 2010; and U.S. Patent 8,048,297, Leach et al., issued November 1, 2011. A device, such as the GPS™ Platelet Concentrate System, commercially available from Biomet Biologics, LLC (Warsaw, Indiana, USA), may be used to isolate adipocytes. Separators and methods that may be used to fractionate BMA at steps 610 and 615 are also described, for example, in U.S. Application Publication 2006/0278588, Woodell-May, published December 14, 2006. The BMA may be positioned in a separator according to various embodiments in step 610. Once the BMA is positioned in the separator, a selected fraction of the BMA may be separated from the BMA in step 615.

Once the BMA is placed in the separator, separator is spun in a centrifuge in a range between about 1,000 and about 8,000 RPM. This produces a force between about 65 and about 4500 times greater than the force of normal gravity, as generally calculated in the art, on the separator and the BMA. At this force, the more dense material in a BMA sample is forced toward the bottom end of the tube. The separator can thus be used to remove nucleated cells from the bone marrow sample. In various embodiments, concentrated BMA has a concentration of nucleated cells that is at least 2, at least 3, at least 4, or at least 5 times the concentration of nucleated cells in BMA.

Obtaining a Cytokine cell suspension from Blood Clots

In other embodiments comprising the use of blood, a liquid comprising cytokine-producing cells may be trapped in a blood clot. Cell releasate can be generated from the blood clot by either compression ("squeezing"), clot disruption, or centrifugation. The blood clot can be made with or without anticoagulant and with or without exogenous thrombin by combining blood or a blood fraction with a clotting agent. Suitable clotting agents include thrombin (e.g., bovine, recombinant human, pooled human, or autologous), autologous clotting protein, and polyethylene glycol. Calcium may be in the form of a calcium salt, such as calcium chloride.

In some embodiments, the clotting agent comprises a clotting protein, which may be a clotting fraction derived from a blood obtained from the patient to be treated. A suitable clotting fraction can be obtained by a process of: loading whole blood or plasma with a calcium solution (e.g., calcium chloride in ethanol) into a blood isolation device; optionally heating the whole blood or plasma for at least about 20 minutes, at a temperature of at least about 20° C; and isolating the clotting fraction. The isolating may be performed by centrifuging the heated whole blood or plasma. A suitable isolation device is commercially available as the Clotalyst™ Autologous Thrombin Collection System (hereinafter "Clotalyst System"), sold by Biomet Biologics LLC, Warsaw, Indiana, USA.

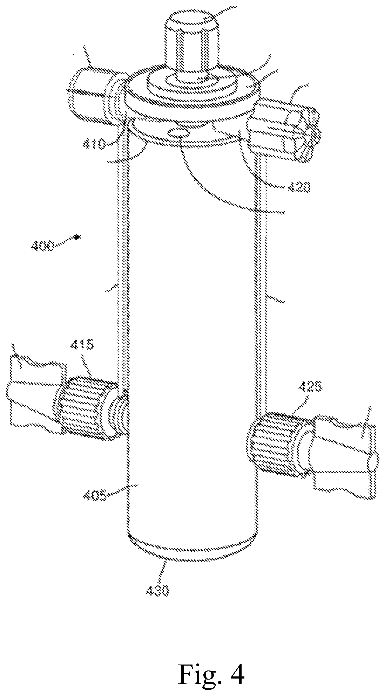

An exemplary procedure for producing a clotting agent using a device 400 of Fig. 4 begins with injecting a reagent comprising calcium chloride and ethanol into the main chamber 405 through the first port 410. Glass beads are also placed in the main chamber 405. After the reagent has been injected, the first port 410 is closed using the first replacement cap 415. Blood with anticoagulant is injected into the main chamber 405 through the second port 420. After the blood has been injected, the second port 420 is closed using the second replacement cap 425. Optionally, the syringes and blood separation device 400 are pre-heated to a temperature of about 25°C.

The contents of the blood component separation device 400 are mixed by repeatedly inverting the device 400, e.g. about twelve times, so as to contact the blood with the glass beads. After mixing, the device is incubated The incubation process can be at a temperature and for a duration that will permit the contents of the device 400 to be heated at about 25°C for about 15 minutes. Upon completion of the incubation period, a clotted mass of red blood cells, blood plasma, and glass beads forms at a second end 406 of the main chamber 405. After incubation is complete, the device 400 is shaken enough to dislodge and break-up any gel that may be present.

Obtaining a Cytokine Suspension Using Non-Centrifugal Methods

As noted above, the liquid containing white blood cells can be obtained by non-centrifugal means, such as by culturing. As referred to herein, a "non-centrifugal method" comprises a process for obtaining tissue fractions comprising cytokine-producing cells from tissue without use of a centrifuge. In some embodiments, methods are "non-gravimetric," wherein, based on physical, chemical or physicochemical properties of the cells other than density, wherein the concentration of white blood cells in the fraction are higher than the concentration of white blood cells in the tissue. Such non-gravimetric methods are, in particular, distinguished from methods wherein a white blood cell fraction is created by centrifugation of whole blood or other tissue. In some embodiments, the non-centrifugal method comprises a process solely based on such properties of white blood cells other than density. Non-centrifugal methods include filtration, antibody binding, and electrophoretic methods.

For example, as discussed above, white blood cells may be prepared from whole blood, bone marrow aspirate or other tissue, using filtration. White blood cells and other cytokine-producing cells obtained from blood, bone marrow, adipose tissue or other sources may also be cultured, using methods among those known in the art. The cells may be then suspended in a suitable medium, such as plasma, so as to maintain their viability and facilitate mixing or other contact with a solid extraction material. A liquid containing the cells may also be produced by compression or disruption of blood clots, as described above.

Contacting a Cytokine cell suspension With an Extraction Material and Isolating a Protein Solution

In further reference to the exemplified process of Fig. 1, the cytokine cell suspension is incubated or otherwise contacted with a solid extraction material (step 140) to produce a protein-containing liquid. This liquid is then isolated (step 150) from the solid extraction material, as a Protein Solution of the present technology. Without limiting the scope, mechanism or function of the present technology, solid extraction materials useful herein concentrate cytokines or other proteins in the liquid volume of cytokine-producing cells and may, in some embodiments, activate, stimulate or otherwise increase production of cytokines, including IL-1ra. Thus, in some embodiments, methods comprising activating a cytokine cell suspension with a solid extraction material.

The solid extraction material can include various materials that provide a particular surface area to contact the cells. The solid extraction material may be a continuous material or may be discontinuous and comprise a plurality of separate particles. For example, the solid extraction material may be in the form of a plurality of beads, fibers, powder, a porous material, or a surface of a container comprising the liquid containing the cells. The solid extraction material may comprise geometric forms having various cross-sectional shapes, such as spherical, oval, or polygonal, among others. The solid extraction material can also comprise a continuous porous network, similar to a sponge, or can include a plurality of individual porous particles. The solid extraction material may also provide a larger surface area by being porous in comparison to a nonporous material.

In some embodiments, the solid extraction material includes particles having a large aspect ratio, for example, where the particles are needle-like in shape. The solid extraction material may also be formed as long fibers and may be or take a form similar to glass wool.

In some cases, the solid extraction material can comprise the internal walls of a container holding the cytokine cell suspension. For example, the solid extraction material may comprise the lumen of a syringe that contains the cytokine cell suspension. Other containers include tubes, such as centrifuge tubes, or a blood fractionation device or concentrator assembly as described elsewhere herein.

Where the solid extraction material is a continuous material, such as a porous sponge-like material, the solid extraction material can be used in an amount sufficient to absorb or adsorb or include substantially the entire liquid volume of cytokine-producing cells within the pores or interstices of the solid extraction material. Where the solid extraction material is a discontinuous material, such as a plurality of particles, the solid extraction material can be combined with the liquid containing the cells to form a slurry-like composition. The slurry can vary in consistency from paste-like, having a high-solids fraction, to a readily flowable slurry having a low-solids fraction.

The solid extraction material can provide a large surface area with which to contact the cells. However, in some cases, the solid extraction material can be further treated to increase its surface area, for example, by physically or chemically etching or eroding the surface of the solid extraction material. With respect to chemical etching, a corrosive agent can be used to modify the surface of the solid extraction material depending on the nature of the material. The modified surface may be produced by employing an alkali or an acid, for example chromosulphonic acid, in particular about 20% to about 80% in strength, preferably about 50% chromosulphonic acid. The solid extraction material can be incubated with the corrosive agent for about 5 min to about 30 min in order to chemically etch the surface and increase the surface area. The solid extraction material can then be washed to remove the corrosive agent. For example, the solid extraction material can include the internal walls of a container for holding the cytokine cell suspension where the internal walls are etched to subsequently increase the surface area in contact with the liquid.

Various polymers, metals, ceramics, and glasses can be used as the solid extraction material. In some embodiments, the solid extraction material comprises a hygroscopic material. Examples of suitable solid extraction material materials include glasses, minerals, polymers, metals, and polysaccharides. Minerals include corundum and quartz. Polymers include polystyrene, polyethylene, polyvinyl chloride, polypropylene, and polyacrylamide. Metals include titanium. Polysaccharides include dextran and agarose. A preferred solid extraction material comprises, or consists essentially of, polyacrylamide, as further described below.

The solid extraction material may comprise, for example, continuous solid extraction material of glass or a plurality of glass particles, glass wool, a continuous solid extraction material of metal such as titanium, a plurality of metal beads, metal powder, and combinations thereof. A continuous solid extraction material of metal can include a block or other three-dimensional shape formed of porous metal or metal alloys with an open cell structure. The solid extraction material may include various beads or particles of various sizes including substantially spherical beads. Beads include polystyrene beads, polyacrylamide beads, glass beads, metal (e.g., titanium) beads, or any other appropriate beads. Beads may be any size appropriate for the container and the amount of cytokine cell suspension being used. In some instances, bead sizes can range from about 0.001 millimeters to about 3 millimeters in diameter. Where the bead size is sufficiently small, the beads can appear more like a powder.

Polyacrylamide beads used as the solid extraction material can be formed by polymerizing acrylamide monomer using controlled and standardized protocols as known in the art to produce relatively uniform beads formed of polyacrylamide gel. In general, polyacrylamide is formed by polymerizing acrylamide with a suitable bifunctional crosslinking agent, most commonly N,N'-methylenebisacrylamide (bisacrylamide). Gel polymerization is usually initiated with ammonium persulfate and the reaction rate is accelerated by the addition of a catalyst, such as N,N,N',N'-tetramethylethylenediamine (TEMED). In various embodiments, polyacrylamide beads comprise 0.5 micromole of carboxyl groups per milliliter of beads, imparting a slight anionic character (negative charge). The beads are also typically resistant to changes in pH, and are stable in many aqueous and organic solutions. By adjusting the total acrylamide concentration, the polyacrylamide gel can be formed in a wide range of pore sizes. Moreover, the polyacrylamide beads can be formed in many sizes and can have relatively uniform size distributions. Bead size may range from several micrometers in diameter to several millimeters in diameter. For example, various types of Bio-Gel™ P polyacrylamide gel beads (Bio-Rad Laboratories, Hercules, California, USA) have particle sizes ranging from less than about 45 $\mu$m up to about 180 $\mu$m. Polyacrylamide beads are also available from SNF Floerger (Riceboro, Georgia, USA), Pierce Biotechnology, Inc. (Rockford, Illinois, USA), and Polymers, Inc. (Fayetteville, Arkansas, USA).

Once polymerized, polyacrylamide beads can be dried and stored in a powder-like form. The dry beads are insoluble in water but can swell considerably upon being rehydrated. Rehydration returns the polyacrylamide beads to a gel consistency that can be from about two to about three times the dry state size. Thus, dry polyacrylamide beads (i.e., desiccating polyacrylamide beads) may be used to absorb a portion of a liquid volume, including solutes smaller than the bead pore size, and can serve to concentrate IL-1ra and other proteins produced by the cytokine-producing cells. For example, combining dry polyacrylamide beads with the blood and/or platelet-rich plasma in step 230 activates production of IL-1ra by the cytokine-producing cells and also reduces the total liquid volume as the dry beads rehydrate and swell.

Without limiting the scope, mechanism or function of the present technology, it has been discovered that surface contact with the solid extraction material can activate the cells and the solid extraction material can, in some cases, assist in the separation and concentration of the resulting Protein Solution rich in cytokines, including IL-1ra. For example, in the case of a porous solid extraction material, a portion of the liquid comprising the cells can enter the pores and remain therein. Cells in the liquid may contact this additional surface area. In some embodiments, the pores are too small for the cells to enter, but a portion of the liquid can enter the pores. Liquid can be removed from the solid extraction material and pores by centrifuging, for example.

The solid extraction material is preferably sterilized, using techniques among known in the art, in order to prevent contamination of the cytokine cell suspension. For example, heat and pressure sterilization methods, such as autoclaving, may be used depending on the particular composition of the solid extraction material. Alternative methods, such as chemical sterilization or irradiation, can be used where the solid extraction material may be adversely affected by the autoclaving process.

In some embodiments, the cytokine cell suspension is incubated with solid extraction material for a time effective to remove a portion of the liquid. The incubation may be carried out over a period from about 30 seconds to about 72 hours and may be carried out at a temperature from about 20°C to about 41°C. For example, the incubation may be 24 hours or less, 10 hours or less, 5 hours or less, 2 hours or less, 1 hour or less, 30 minutes or less, 15 minutes or less 10 minutes or less, 5 minutes or less, 4 minutes or less, 3, minutes or less, or 2 minutes or less. Incubation may be at least about 15 seconds, at least about 30 seconds, at least about 1 minutes, at least about 90 seconds, at least about 2 minutes, at least about 10 minutes, or at least about 30 minutes. In some embodiments, incubation s from about 1 minute to about 3 minutes. In some embodiments the liquid is not incubated, but is contacted with the solid extraction material for only so long as necessary to perform subsequent processing. The contacting may occur at ambient conditions, e.g., at a temperature of about 20-25°C.

In some embodiments, the cytokine cell suspension and the solid extraction material are agitated to more thoroughly mix these components during contact. The agitation may be accomplished by inverting, shaking, rocking, stirring, or vortexing the liquid and solid extraction material. Agitation may increase contact of the cells within the liquid with the solid extraction material. Agitation may be performed once, repeated multiple times, repeated periodically, or may be continuous. The liquid comprising the cells and the solid extraction material may also be agitated while the liquid is stimulated with the electromagnetic field. Additional aspects and features relating to producing protein-rich solutions using polyacrylamide beads and other solid extraction materials are described in: U.S. Patent Application Publication 2009/0220482, Higgins et al., published September 3, 2009; U.S. Patent Application Publication 2010/0055087, Higgins et al., published March 4, 2010; U.S. Patent Application Publication 2011/0052561, Hoeppner, published March 3, 2011; International Application Publication WO 2012/030593, Higgins et al., published March 8, 2012; and U.S. Patent Application Publication 2012/0172836, Higgins et al., published July 5, 2012. Compositions and methods useful in aspects of the present technology are also described in the following applications: U.S. Patent Application No. 13/840,562, Binder et al., Methods and Non-Immunogenic Compositions for Treating Inflammatory Diseases; U.S. Patent Application 13/841,083, Landrigan, et al., Treatment of Inflammatory Respiratory Disease Using Protein Solutions; U.S. Patent Application 13/837,480, O'Shaughnessey, et al., Treatment of Pain Using Protein Solutions; U.S. Patent Application 13/839,280, Leach et al., Methods for Making Cytokine Compositions from Tissue Using Non- Centrifugal Methods; U.S. Patent Application 13/840,129, Matusuka, et al., Treatment of Collagen Defects Using Protein Solutions; and U.S. Patent Application 13/841,103, Landrigan, et al., Treatment of Peripheral Vascular Disease Using Protein Solutions.

Contacting of the cytokine cell suspension with the solid extraction material may be performed using a suitable container or other apparatus to effect the contact. Contacting may be performed in a continuous process wherein a flow of the liquid is passed over or through the solid extraction material, or the liquid and solid extraction material may be contained in a vessel. As discussed above, the vessel may comprise the solid extraction material, or may merely serve as a container holding the beads or other forms of the material. Containers useful in the present technology include those known in the art, such as the Plasmax™ Plus Plasma Concentrator, commercially available from Biomet Biologies, LLC (Warsaw, Indiana, USA) and may include those devices and methods of use as described in U.S. Patent No. 7,553,413, Dorian et al., issued June 30, 2009; and U.S. Patent. 7,694,828, Swift et al., issued April 13, 2010.

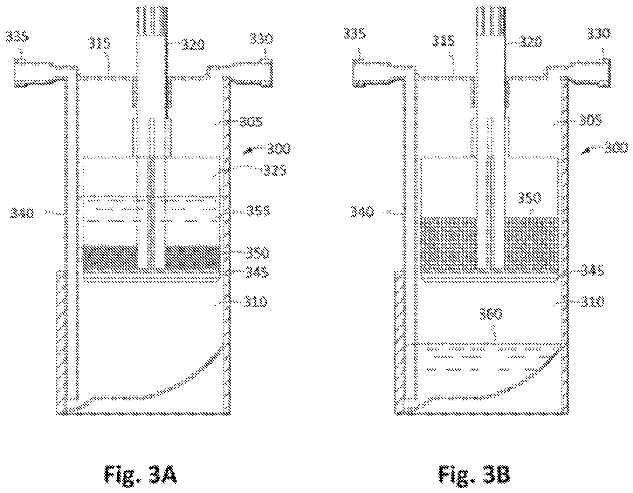

Such a device is shown in Figs. 3A and 3B, for exemplary use with a polyacrylamide gel bead solid extraction material. The device 300 has an upper chamber 305 and a lower chamber 310. The upper chamber 305 has an end wall 315 through which the agitator stem 320 of a gel bead agitator 325 extends. The device 300 also has an inlet port 330 that extends through the end wall 315 and into the upper chamber 305. The device 300 also includes an outlet port 335 that communicates with a plasma concentrate conduit 340. The floor of upper chamber 305 includes a filter 345, the upper surface of which supports desiccated concentrating polyacrylamide beads 350.

During use, a fluid 355 containing cytokine-producing cells and, optionally, platelets is injected to the upper chamber 305 via the inlet port 330 and mixed with the polyacrylamide beads 350. The fluid 355 and polyacrylamide beads 350 may be mixed by rotating the agitator stem 320 and the gel bead agitator 325, to help mix the fluid 355 and beads 350. The mixed fluid 355 and polyacrylamide beads 350 are then incubated for the desired time at the desired temperature. The device 300 is then centrifuged so that liquid passes to the lower chamber 310 while the polyacrylamide beads 350 are retained by a filter 345, thereby separating the polyacrylamide beads 350 from the resulting solution 360 of IL-1ra and other proteins that collects in the lower chamber 310. The solution 360 may be removed from the device via outlet port 335.

In some embodiments, a Protein Solution can be made in a process wherein a cytokine cell suspension is isolated from a tissue and then contacted with a solid extraction material in a continuous process. Referring again to Fig. 1, in some embodiments the isolating 110, 120, 135 and contacting 140 are performed using a single apparatus, referred to herein as a single separation and concentration device ("S/C device"). One such device is described in U.S. Patent Application Publication 2013/0259951, O'Connell, published October 3, 2013.

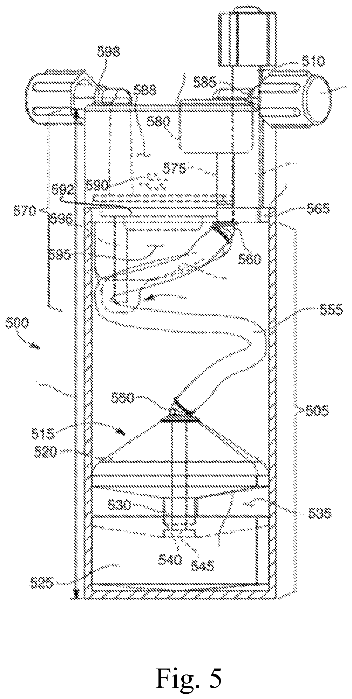

The S/C device comprises a separation region, a first concentration region, a second concentration region, a buoy system, an inlet port, a check valve, a first withdrawal port and a second withdrawal port. Fig. 5 shows an S/C device 500 capable of generating an anti-inflammatory cytokine composition from whole blood. For example, the method may start with obtaining a volume of whole blood, which is filled into a separation region 505 of the S/C device 500 by injecting through the inlet port 510. A buoy system 515 is located within the separation region 505. The buoy system comprises a first buoy member 520, a second buoy member 525, and a third buoy member 530 that couples the first buoy member 520 to the second buoy member 525. A space between the first and second buoy members 520, 525 defines a buoy separation region 535. A density of each buoy member can be selected depending on what blood fraction is desired as a result of a separation. The buoy system 515 can include a selected buoy system, such as the buoy system generally used in the GPS® II or GPS ®III gravity platelet separation system sold by Biomet Biologics, LLC. (Warsaw, Indiana, USA). Buoy systems are disclosed in: U.S. Patent 7,845,499, Higgins et al., issued December 7, 2010; U.S. Patent 7,806,276, Leach et al., issued October 5, 2010; and U.S. Patent 7,992,725, Leach et al., issued August 9, 2011.

A method for obtaining a Protein Solution comprises spinning the S/C device 500 by centrifugation. Centrifugal forces allow the buoy system 515 to move through the whole blood, resulting in a fraction of the whole blood to be located in the buoy separation region 535. For example, this fraction may comprise platelet-rich plasma. With a use of a withdrawal syringe, the selected fraction can be removed from the collection volume 535 through the third buoy member 530 that defines a removal passage 540 that is connected with collection face passages 545. A connection elbow 550 can interconnect with the removal passage 540 to allow a vacuum to be formed through the connection elbow 550, the collection passage 540, and the buoy collection passages 545. A collection tube 555 can interconnect the connection elbow 550 with a withdrawal elbow 560 that extends from a wall 565 that can be a bottom wall of concentration region 570. A second withdrawal tube 575 can be first connected with a check valve assembly 580 and a first withdrawal port 585. The first withdrawal port 585 can be connected with the withdrawal syringe with a Luer lock type connection or other appropriate connection.

The check valve assembly 580 ensures the fraction being removed flows in one direction and prevents the fraction being removed from reentering the second withdrawal tube 575. Furthermore, when material is pushed back into the check valve assembly 580 from the first withdrawal port 585, such that material will enter the concentration region 570, a disc within the check valve 580 can flex down towards the second withdrawal tube 575 and close an opening and thereby open a second opening within the check valve assembly 580. The second opening allows the fraction to be pushed into the concentration region 570.

Therefore, the blood fraction is then re-injected through the first withdrawal port 285, through the check valve assembly 580, and into an upper volume 588 of the concentration region 570. Polyacrylamide beads 590 are added to the blood fraction in the upper volume 588 and the blood fraction and the polyacrylamide beads 590 can be mixed by shaking. Optionally, the blood fraction and the beads 590 can be incubated for a selected period of time before proceeding with the method.

The method comprises a second step of spinning by centrifugation. During the second centrifugation, the anti-inflammatory cytokine composition is separated from the beads 590 by being forced through a filter 592 and into a lower concentration region 595 of the concentration region 570. The Protein Solution can be withdrawn through a third withdrawal tube 596 and out a second withdrawal port 598 by use of a second withdrawal syringe. Again, the syringe can be connected to the second withdrawal port by a Luer® lock type connection.

Referring again to Fig. 1, following contacting the liquid with the solid extraction materials, a Protein Solution is isolated, as indicated at step 150. Isolation may be accomplished by drawing off at least a portion of the liquid volume and leaving the beads. In some cases, the extraction material may be sedimented by centrifugation prior to drawing off the Protein Solution. Isolation may also be performed by filtration, where the material is retained by a filter and the Protein Solution passes through the filter using centrifugal force or by using vacuum, for example. If the incubation with extraction material utilizes dry polyacrylamide beads, the liquid volume may be reduced as the beads swell upon rehydration, thereby concentrating the resulting Protein Solution. To maintain the increased concentration, care should be taken in the isolation step so as to avoid compressing the beads or drawing liquid out from the swollen beads. For example, high centrifugal force or high vacuum may collapse the beads and/or draw liquid out of the internal volume of the beads.

Optional Electromagnetic Stimulation

The cytokine cell suspension can be stimulated with an electromagnetic field, before or during the contacting of the liquid with a solid extraction material. Thus, in some embodiments, stimulation of the liquid comprising the cells can be performed prior to contacting the liquid and the solid extraction material. However, it is preferred that at least a portion of the contacting step and at least a portion of the stimulating step overlap in time such that the liquid comprising the cells is concurrently in contact with the solid extraction material and stimulated with the electromagnetic field.

Stimulating the cytokine cell suspension with an electromagnetic field may involve various forms of electromagnetic stimulation, such as a pulsed electromagnetic field or a capacitively coupled electromagnetic field. In some embodiments, the liquid is stimulated using a power source coupled to a stimulation coil. The current passing through the coil produces a pulsing magnetic field which induces in the liquid a pulsing electric field. The coil may partially surround the liquid as it is held within a container, such as a tube or syringe. The coil may be integrated into to the container holding the cytokine cell suspension or may be removable. For example, a plastic tube can be formed with an integrated coil or the coil can be temporarily coupled to the container or placed within the container; for example, the tube can be configured so that the coil can be snapped onto the container. The power source can be coupled to the coil as needed to perform the stimulating step.

Stimulation of the liquid with an electromagnetic field may also include placing at least two electrodes across the liquid. Electrical energy may then be applied to the electrodes so as to capacitively couple the electrodes and generate the electromagnetic field there between. The electromagnetic field is therefore able to pass through the liquid so as to increase the rate and/or amount of cytokine production. In other embodiments, electrodes can be used to produce a direct current or one or more coils can be used to produce a pulsed electromagnetic field.

The strength of the electromagnetic field during stimulation can be at least about 0.5 microvolts per centimeter, whether produced by direct current, capacitively coupled current, or pulsed electromagnetic field. In the case of a direct current electrode, the amplitude of the current can be from about 1 to about 200 microamperes, and in some embodiments, the amplitude may be from about 20 to about 100 microamperes. In still further embodiments, the current may be about 20, about 60, or about 100 microamperes. It should be understood, however, that the amplitude of the current may be of other suitable magnitudes.

The electromagnetic field applied during the stimulating step may be constant or vary over time. For example, a sinusoidal time varying electromagnetic field can be applied using the electrodes placed across the liquid. Such a sinusoidal time varying electromagnetic field can have a peak voltage across the electrodes from about 1 volt to about 10 volts, and in some embodiments, the peak voltage can be about 5 volts. The corresponding electric field produced can have an amplitude of from about 0.1 millivolt per centimeter (mV/cm) to about 100 mV/cm, and in some embodiments can be about 20 mV/cm. The sinusoidal time varying electric field may have a frequency of from about 1,000 Hz to about 200,000 Hz, and in some embodiments the frequency may be about 60,000 Hz.

The electromagnetic field applied to the liquid may also be a pulsed electromagnetic field. The pulsed electromagnetic field can be induced using an external coil and a pulse generator. In this regard, a pulsed electromagnetic field may have a pulse duration of from about 10 microseconds per pulse to about 2000 microseconds per pulse. The pulse duration in one embodiment can be about 225 microseconds. The pulses may include electromagnetic bursts, in which a burst can comprise from 1 pulse to about 200 pulses. Alternatively, the electromagnetic field may have bursts that comprise from about 10 pulses to about 30 pulses. In this regard, in one embodiment each burst may comprise about 20 pulses.

The frequency at which bursts in the pulsed electromagnetic are applied may vary. In this regard, bursts can be repeated at a frequency of from about 1 Hz to about 100 Hz in some embodiments, and can be repeated at a frequency of about 10 Hz to about 20 Hz in other embodiments. Furthermore, bursts can repeat at a frequency of about 1.5 Hz, about 15 Hz or about 76 Hz. A burst can have a duration from about 10 microseconds up to about 40,000 microseconds. In this regard, a burst can have a duration of about 4.5 milliseconds.

Suitable devices for generating a capacitively coupled electromagnetic field include SpinalPak® spinal stimulator (EBI, L.P., Parsippany, New Jersey) or a DC stimulation device such as an SpF® XL IIb spinal fusion stimulator (EBI, L.P., Parsippany, New Jersey). Pulsed electromagnetic fields can be produced using various known methods and apparatuses, such as using a single coil or a pair of Helmholtz coils. For example, a suitable apparatus includes the EBI Bone Healing System® Model 2001 (EBI, L.P., Parsippany, New Jersey) and the BTBS stimulation coil. With respect to direct current, an electric field may be generated using any known device for generating a direct current electric field, such as for example, the Osteogen™ implantable bone growth stimulator (EBI, L.P., Parsippany, New Jersey). Other suitable devices for generating electromagnetic fields may be used.

Electromagnetic stimulation of the cytokine cell suspension can be continued and/or repeated as desired with respect to contacting the liquid and the solid extraction material. It should be understood, however, that the step of stimulating the liquid with an electromagnetic field includes fields other than, or in addition to, electric or electromagnetic fields associated with ambient conditions (such the electromagnetic fields generated by casual exposure to radios, telephones, desktop computers or similar devices).

In some embodiments, both the contacting and stimulating steps as shown in Fig. 1 are performed in less than about 1 hour. The contacting and stimulating steps can also be performed at temperatures ranging from about 20°C to about 37°C. In a preferred embodiment, the temperature of the cytokine cell suspension is kept at about 37°C during the contacting and stimulating steps. One or both of the contacting and stimulating steps are typically performed ex vivo.

Other Methods for Forming Protein Solutions

The present technology provides other methods for forming Protein Solutions, such as the admixture of proteins and other components and the isolation and concentration of proteins and components without using solid extraction materials. Protein Solutions of the present technology can be made entirely comprising proteins made by such methods, or by addition of proteins made by such methods with components or solutions made by tissue isolation and processing with solid extraction materials, as described above.

For example, various methods provide acellular or substantially acellular Protein Solutions, comprising one or more proteins as described above. Without limiting the scope, mechanism or function of the present technology, such acellular anti-inflammatory cytokine compositions may offer advantages in certain applications, insofar as they may not create an immunogenic response in subjects to whom they are administered.

In particular, by way of example, a Protein Solution may comprise interleukin-1 receptor antagonist (IL-1ra) that is synthetic or recombinant, or isolated from autologous, allogeneic or xenogeneic blood or other biologic sources, aside from the methods described above. For example, Kineret™ (anakinra) is a recombinant, non-glycosylated form of IL-1ra, sold by Amgen Manufacturing, Ltd. (Thousand Oaks, California). Various recombinant interleukin-1 inhibitors and methods of treatment are described in U.S. Patent 6,599,873, Sommer et al., issued July 29, 2003; U.S. Patent 5,075,222, Hannum et al., issued December 24, 1991; and U.S. Application Publication 2005/0197293, Mellis et al., published September 8, 2005. In addition, methods for producing IL-1ra from body fluids, including the use of autologous fluids, are described in U.S. Patent 6,623,472, Reincke et al., issued September 23, 2003; U.S. Patent 6,713,246, Reinecke et al., issued March 30, 2004; and U.S. Patent 6,759,188, Reinecke et al., issued July 6, 2004. When an allogeneic anti-inflammatory cytokine composition is to be generated, multiple sources of IL-1ra from multiple subjects may be pooled together.

More generally, methods for making acellular Protein Solutions can comprise culturing cells in a cell culture that either naturally produce anti-inflammatory cytokines, such as IL-1ra, or cells that are engineered to produce such cytokines. Non-limiting examples of cells that naturally produce anti-inflammatory cytokines include adipose tissue cells, adipocytes, adipose-derived stem cells, stromal cells, bone marrow cells, mesenchymal stem cells, and blood cells.

In various embodiments, cell lines can be engineered to overproduce an anti-inflammatory cytokine. Non-limiting examples of anti-inflammatory cytokines include VEGF, TNF-$\alpha$, IL-1ra, sTNF-RI, sTNF-RII, PGDF-AB, PDGF-BB, IGF-I, EGF, TGF-$\beta$1, sIL-1RII, and HGF. Stable eukaryotic cell lines can be generated that overexpress an anti-inflammatory cytokine by transfecting eukaryotic cells, such as mammalian cells, with recombinant DNA comprising a gene encoding an anti-inflammatory cytokine and a selectable marker. Alternatively, prokaryotes and yeast can be engineered to overexpress an anti-inflammatory cytokine by transformation with recombinant DNA comprising a gene encoding an anti-inflammatory cytokine and a selectable marker. Transformations and transfections can be performed with recombinant DNA molecules comprising a DNA sequencing encoding an anti-inflammatory cytokine, such as IL-1ra, and a selectable marker. Eukaryotic and prokaryotic cells can be engineered to overexpress the anti-inflammatory cytokine constitutively or by induction. Methods for expressing anti-inflammatory cytokines, such as IL-1ra, sTNF-RI, and sTNF-RII, and sILI-RII in eukaryotic and prokaryotic cells are described in U.S. Patent 6,337,072, Ford et al., issued January 8, 2002; and U.S. Application Publication 2001/0053764, Sims et al., published December 20, 2001.

When a IL-1ra gene is transcribed in humans, the mRNA can be spliced into four variants, resulting in four isoforms of translated IL-1ra. SEQ ID NOs: 1, 3, 5, and 7 are the cDNAs for IL-1ra isoforms 1-4 respectively, and SEQ ID NOs: 2, 4, 6, and 8 are the amino acid sequences of IL-1ra isoforms 1-4 respectively. Collectively, the IL-1ra isoforms are referred to as "IL-1ra." SEQ ID NO: 9 is the cDNA sequence for sTNF-RI and SEQ ID NO:10 is the amino acid sequence for sTNF-RI. SEQ ID NO:11 is the cDNA sequence for sTNF-RII and SEQ ID NO:12 is the amino acid sequence for sTNF-RII. SEQ ID NO:13 is the cDNA sequence for sIL-1RI and SEQ ID NO:14 is the amino acid sequence for sIL-1RI. SEQ ID NOs 15 and 17 are the cDNAs for sIL-1RIIv1 and sIL-1RIIv3 respectively, and SEQ ID NOs:16 and 18 are the amino acid sequences for sIL-1RIIv1 and sIL-1RIIv3 respectively. The cDNA sequence for IL-1RIIv2 is a non-coding sequence; therefore, it is not included.

To express either IL-1ra, sTNF-RI, or sTNF-RII (generically referred to as a "protein of interest") in a prokaryotic culture, for example in a particular bacteria, a cDNA sequence (SEQ ID NOs: 1, 3, 5, 7, 9, 11, 13, 15, or 17) is cloned into an expression vector suitable for the bacteria. The expression vector should comprise a strong promoter, and a selectable marker, such as antibiotic resistance. Non-limiting examples of antibiotics capable of killing bacteria cells include ampicillin, tetracycline, kanamycin, and chloramphenicol. The expression vector should further comprise elements that result in constitutive or inducible expression of the protein of interest. Optionally, a DNA sequence corresponding to a tag functionally coupled to the protein of interest that allows for identification and purification of the protein can be present in the vector adjacent to the gene for the protein of interest. For example, an N or C-terminal His tag can be used to detect proteins with anti-His antibodies, and they allow for purification on nickel columns. When the expression vector comprising a gene expressing a protein of interest is prepared, a bacteria cell, for example E. coli, can be transformed with the expression vector. The selectable marker ensures that only cells transformed with the vector will survive in LB broth supplemented with an antibiotic corresponding to the selectable marker. The bacteria can then be grown in LB broth supplemented with the antibiotic for expression and purification. Expression vectors, methods for cloning a protein of interest into an expression vector, methods for transforming prokaryotic cells, methods for expressing protein from transformed prokaryotic cells, and protein purification methods are commonly known by those with ordinary skill in the art.

To express a protein of interest in a eukaryotic culture, for example in mammalian cells, a cDNA sequence (SEQ ID NOs: 1, 3, 5, 7, 9, 11, 13, 15, or 17) is cloned into an expression vector suitable for a particular mammalian cell. The expression vector should comprise a strong promoter, and a selectable marker, such as antibiotic resistance. Non-limiting examples of antibiotics capable of killing mammalian cells include geneticin and gentamicin. The expression vector should further comprise elements that result in constitutive or inducible expression of the protein of interest. Optionally, a DNA sequence corresponding to a tag functionally coupled to the protein of interest that allows for identification and purification of the protein can be present in the vector adjacent to the gene for the protein of interest. When the expression vector comprising a gene expressing a protein of interest is prepared, a mammalian cell, such as a human cell, can be transfected with the expression vector. Transfected cells can be grown in a cell culture medium supplemented with an antibiotic corresponding to the selectable marker. The presence of the antibiotic allows for the isolation of stable cell lines. Stable cell lines can then be grown in cell culture medium supplemented with antibiotic for expression and purification. Expression vectors, methods for cloning a protein of interest into an expression vector, methods for transfecting eukaryotic cells and developing stable cell lines, methods for expressing protein from transfected eukaryotic cells, and protein purification methods are commonly known by those with ordinary skill in the art.

Alternatively, eukaryotic cells that have not been genetically altered by DNA transfection can be cultured. The eukaryotic cells can be primary cultures, i.e., cells grown directly from a eukaryotic donor, such as a human, or the eukaryotic cells can be established cell lines. Many established cell lines are available commercially from American Type Culture Collection, Inc. (Manassas, VA, USA). The cells can be grown with or an exogenous signal, such as a recombinant protein. Eukaryotic cells are often cultured in culture flasks with cell culture medium. The cell culture medium can be recovered from the flasks, and centrifuged to remove any non-adherent cells.

A cell culture can be a monolayer culture, a non-adherent culture, or a bioreactor. A monolayer culture comprises anchorage-dependent cells that are cultured on a suitable substrate that allows cell adhesion and spreading, such as cell culture flasks and cell culture dishes. A non-adherent culture comprises cells that are maintained in a suspension. Suitable cells are either not anchorage-dependent, or they are anchorage-dependent cells that have been adapted for culture in a suspension. Many cell lines, for example many insect cells, can be grown in either a monolayer or a suspension. A bioreactor is a device that can support a biologically active environment in which chemical processes are carried out and/or biochemically active substances are derived. Bioreactors can include suspended or immobilized cells. Monolayer cultures, non-adherent cultures, and bioreactors can be maintained by methods commonly used in the art.

In some embodiments, the cell culture is subjected to an electromagnetic field, so as to stimulate the production of one or more proteins. Stimulating the culture with an electromagnetic field may involve various forms of electromagnetic stimulation, such as a pulsed electromagnetic field or a capacitively coupled electromagnetic field. Methods and conditions for stimulation include those discussed above.

Cell cultures can either release anti-inflammatory cytokines into culture medium naturally, or the cultures can be induced to release the anti-inflammatory cytokines into the culture medium. The culture medium can be isolated by aspiration, centrifugation or filtration to form the acellular anti-inflammatory cytokine composition.

In some embodiments, an anti-inflammatory cytokine is isolated from urine, for use in producing a Protein Solution of the present technology. Proteins can be isolated from urine by methods among those known in the art. One such method is employed in the ProteoSpinTM Urine Protein Concentration Maxi Kit sold by Norgen Biotek Corp. (Thorold, Ontario, Canada). This kit utilizes an ion exchange resin integrated into a spin column. Briefly, a urine sample is obtained and its pH adjusted to 3.5. The urine is then transferred to a spin column containing the ion exchange resin, which is placed in a collection tube. The column is then centrifuged, wherein the proteins attach to the resin, and the remaining fluids and salts flow into the collection tube and are discarded. The proteins are then washed by applying supplied column activation and wash buffer followed by centrifugation. The flow through is discarded and the wash procedure is repeated. An elution buffer (10 mM sodium phosphate, pH 12.5) is added to the column and neutralizer is added to an elution tube. The spin column containing the elution buffer is placed in the elution tube and centrifuged, whereby the proteins are eluted and captured in the elution tube containing neutralizer.

Therapeutic Compositions

The present technology also provides compositions comprising a Protein Solution and a second component comprising active materials, physiological carriers, and combinations thereof. In various embodiments, the compositions comprise an efficacy ratio as set forth below. In some embodiments, compositions comprise a safe and effective amount of the Protein Solution and a safe and effective amount of a second active. A "safe and effective" amount of a component is an amount that is sufficient to have the desired therapeutic effect in the human or other mammalian subject, without undue adverse side effects (such as toxicity, irritation, or allergic response), commensurate with a reasonable benefit/risk ratio when used in the manner of this technology. The specific safe and effective amount of the component will, obviously, vary with such factors as the particular condition being treated, the physical condition of the patient, the nature of concurrent therapy (if any), the specific components used, the specific route of administration and dosage form, the carrier (if any) employed, and the desired dosage regimen.

Active materials among those useful herein include biologics and pharmaceutical actives. Biologics include blood fractions, such as PRP, blood products, and concentrated bone marrow aspirate (cBMA).

Accordingly, in some embodiments, the present technology provides compositions comprising a safe and effective amount of a Protein Solution and a safe and effective amount of cBMA. cBMA can include hematopoietic, stem cells, stromal stem cells, mesenchymal stem cells, endothelial progenitor cells, red blood cells, white blood cells, fibroblasts, reticulocytes, adipose cells, or endothelial cells. As described above, the Protein Solution may be made using bone marrow aspirate as a cytokine containing tissue. However, a therapeutic composition may additionally comprise cBMA with Protein Solution. In one embodiment, a therapeutic composition comprises a Protein Solution and cBMA in an Protein Solution:cBMA ratio of about 1:1, about 1:2, about 1:3, about 1:4, about 1:5, about 1:6, about 1:7, about 1:8, about 1:9 or about 1:10. Alternatively, the Protein Solution:cBMA ratio can be about 2:1, about 3:1, about 4:1, about 5:1, about 6:1, about 7:1, about 8:1, about 9:1 or about 10:1. The cBMA and Protein Solution may also be produced simultaneously. Thus, in reference to Fig. 1 and the processes described above, bone marrow aspirate may be added to the whole blood obtained in step 115, prior to or during the contacting with a solid extraction material in step 140; such a process involves operation of both steps 115 and 130. For example, bone marrow aspirate may be added to whole blood prior or during isolation of platelet-rich plasma in step 120. Such methods include those described in U.S. Application Publication 2006/0278588, Woodell-May, published December 14, 2006.

In some embodiments, the cBMA and Protein Solution may be produced simultaneously. Thus, in reference to Fig. 1 and the processes described above, bone marrow aspirate may be added to the whole blood obtained in step 115, prior to or during the contacting with a solid extraction material in step 140; such a process involves operation of both steps 115 and 130. For example, bone marrow aspirate may be added to whole blood prior or during isolation of platelet-rich plasma in step 120. Such methods include those described in U.S. Application Publication 2006/0278588, Woodell-May, published December 14, 2006.

Pharmaceutical actives among those useful herein include herein include organic molecules, proteins, peptides, peptido mimetic s, nucleic acids, nucleoproteins, antisense molecules, polysaccharides, glycoproteins, lipoproteins, carbohydrates and polysaccharides, botanical extracts, and synthetic and biologically engineered analogs thereof, living cells (other than cytokine-producing cells) such as chondrocytes, bone marrow cells, viruses and virus particles, natural extracts, and combinations thereof. Specific non-limiting examples of bioactive materials include hormones, antibiotics and other anti-infective agents, hematopoietics, thrombopoietics, antiviral agents, antitumor agents (chemotherapeutic agents), antipyretics, analgesics, anti-inflammatory agents, antiallergy agents, vasodilators, cytokines, growth factors, gene regulators, vitamins, minerals and other nutritionals, nutraceuticals and combinations thereof. In some embodiments, compositions may comprise growth factors in addition to those present in the Protein Solution, such Platelet-Derived Growth Factor (PDGF), Transforming Growth Factor Beta (TGF-β), Insulin-Like Growth Factor (IGF), Fibroblast Growth Factor (FGF), Epidermal Growth Factor (EGF), Vascular Endothelial Growth Factor (VEGF), and Bone Morphogenetic Proteins (BMPs).

The compositions may comprise a carrier material, in addition to any liquid comprising the Protein Solution. It should be understood that in various embodiments of the present technology, methods of treatment employ the Protein Solution as comprised and made above, without further carrier, by direct injection or other application to the site of treatment. However, in other embodiments, an additional carrier material may be used for such reasons as for ease of administration, to facilitate administration using a particular delivery device, enhancing activity, an increasing the length of time the Protein Solution remains at the site of administration. Carriers among those useful herein include saline, hyaluronic acid, collagen, buffers (such as Hank's Buffer), cell culture media, blood products (such as PRP and platelet poor plasma), and mixtures thereof.

Protein Solutions, and compositions comprising Protein Solutions, may be sterilized prior to administration, by any suitable method. For example, a Protein Solution may be sterilized by including a sterile filter to process the product made by the processes described above. In some embodiments, an antibiotic may be included in the solid extraction material during the contacting step described above, or may be added at one or more of the various steps in the methods and treatments described herein. Alternatively, or in addition, the Protein Solution may be produced aseptically.

Protein Solutions and compositions comprising Protein Solutions may also be lyophilized (freeze drying or cryodesiccation) after production, using methods among those known in the art. Thus, as depicted in Fig. 1, the Protein Solution can be lyophilized after it is isolated from the solid extraction material. When freeze dried, the anti-inflammatory cytokine composition can be hydrated with a suitable media 170, at a time before administration or at a time of administration. Hydration may be accomplished by mixing the composition with a solution including saline, buffers, blood, blood fractions, bone marrow aspirate, concentrated bone marrow aspirate, and combinations thereof.

The present technology also provides compositions comprising components derived from blood or other tissue that are suitable for allogeneic administration. In particular, such compositions may comprise proteins and other components isolated from a mammalian subject, or a plurality of mammalian subjects, other than the subject to whom the composition is to be administered in a method of this technology. In further reference to Fig. 1, compositions made by contacting a cytokine cell suspension with a solid extraction material may be made suitable for allogeneic administration by freeze drying, as depicted in step 160, after isolation of the Protein Solution from the solid extraction material. In some embodiments, the composition can be processed to remove cytokine-producing cells present in the Protein Solution composition after contacting step 140. Methods for removing cytokine-producing cells include those known in the art, including filtering, clotting, and gravimetric methods. In some embodiments, isolating the blood fraction comprising plasma and removing cytokine-producing cells are performed essentially simultaneously. Thus, the present technology provides methods for making a non-immunogenic anti-inflammatory cytokine composition, comprising:

(a) obtaining a cytokine cell suspension from a mammalian donor;
(b) contacting the liquid with solid extraction material to generate a composition rich in interleukin-1 receptor antagonist;
(c) removing cytokine-producing cells from the composition; and
(d) freeze drying the composition to produce the non-immunogenic anti-inflammatory cytokine composition.

In some embodiments, a cryopreservative storage solution is added to the Protein Solution, to provide stability for subsequent storage at reduced temperatures. Suitable storage solutions include those in the art, such as glycerol and dimethylsulfoxide (DMSO). The composition may be stored at reduced temperatures, such as from about 1 °C to about 6 °C. In some embodiments, the composition is stored under liquid nitrogen, at about - 80 °C. Preferably, the cryopreservative storage solution is removed from the Protein Solution prior to administration to a mammalian subject. Removal of the storage solution may be performed by methods including those known in the art for processing stored blood comprising cryopreservatives. Washing may be performed using a wash solution, such as saline. In such embodiments, the blood type of the subject to be treated may be matched to the blood type of the donor from whom the cytokine cell suspension was obtained.

Determining Cytokine Concentrations and Calculating Efficacy Ratios

As discussed above, the present technology provides various methods for relating to the determination of whether a device or anti-inflammatory composition production method generates therapeutic levels of an anti-inflammatory composition, including methods for generating anti-inflammatory compositions, and methods for determining whether an anti-inflammatory composition is suitable for treating an inflammatory disorder, using an Efficacy Ratio, as shown in Equation 1.

Equation 1:

$$\text{Efficacy Ratio} = \frac{\left(\frac{[\text{anti} - \text{inflammatory cytokine}]}{[\text{inflammatory cytokine}]}\right) \text{Output}}{\left(\frac{[\text{anti} - \text{inflammatory cytokine}]}{[\text{inflammatory cytokine}]}\right) \text{Input}}$$

Such methods for using Efficacy Ratios include those described further, below.

In Equation 1, denominator is a concentration ratio of anti-inflammatory cytokine to inflammatory cytokine in the biological material before being processed in the device. Therefore, the denominator represents a concentration ratio in the device input. In contrast, the numerator of Equation 1 is a concentration ratio of anti-inflammatory cytokine to inflammatory cytokine in the anti-inflammatory composition generated by the device. The numerator represents a concentration ratio in the device output. Additionally, the brackets ([ ]) in Equation 1, and in the subsequent equations, refer to the concentration of the molecule within the brackets.

Determining the input and output concentrations of inflammatory cytokines and anti-inflammatory cytokines can be performed by any method suitable for measuring such concentrations, including such methods known in the art. Non-limiting examples of methods for determining the concentration of the cytokines include western blotting, enzyme-linked immunosorbent assays (ELISA), high performance liquid chromatography (HPLC), and liquid chromatography-mass spectroscopy (LCMS), surface plasmon resonance (SPR) based techniques (such as with an OCTET® instrument from Forte Bio, Menlo Park, CA), and radioimmunoassay (RIA) techniques.

When determining an Efficacy Ratio, the anti-inflammatory cytokine and the inflammatory cytokine may constitute a cytokine pair. As referred to herein, a "cytokine pair" includes at least one anti-inflammatory cytokine and at least one inflammatory cytokine, in which the anti-inflammatory cytokine mediates the activity of the inflammatory cytokine. Preferably, the anti-inflammatory cytokine inhibits the activity of the inflammatory cytokine. The anti-inflammatory cytokine can antagonize the inflammatory cytokine by preventing the inflammatory cytokine from activating its receptor. The anti-inflammatory cytokine can be a competitive antagonist, a non-competitive antagonist, or an uncompetitive antagonist of the inflammatory cytokine. For example, the anti-inflammatory cytokine can prevent the inflammatory cytokine from activating its receptor by binding to the receptor without activating the receptor, which prevents the inflammatory cytokine from binding to the receptor, or by binding to the inflammatory cytokine, which prevents the inflammatory cytokine from binding to its receptor. It is understood that antagonism can be accomplished by the anti-inflammatory cytokine by alternative routes, for example, by inhibiting signal molecules downstream of the inflammatory cytokine's receptor.

In some embodiments, the inflammatory cytokine may be identified relative to a specific disorder to be treated with a Protein Solution. For example, if an anti-inflammatory composition is to be used to treat inflammation associated with elevated levels of IL-1, then the Efficacy Ratio is referred to as an "Anti-IL-1 Efficacy Ratio" and the anti-inflammatory cytokine in Equation 1 is IL-1ra or IL-1RII, and the inflammatory cytokine is IL-1$\alpha$ or IL-1$\beta$. Likewise, if an anti-inflammatory composition is to be used to treat inflammation associated with elevated levels of TNF$\alpha$, then the Efficacy Ratio is referred to as an "Anti-TNF$\alpha$ Efficacy Ratio" and the anti-inflammatory cytokine in Equation 1 can be sTNF-RI or sTNF-RII and the inflammatory cytokine is TNF$\alpha$. Example Anti-IL-1 Efficacy Ratios are shown in Equations 2 and 3.

$$\text{Equation 2: Anti-IL-1 Efficacy Ratio} = \frac{\left(\frac{[\text{IL}-1\text{ra}]}{[\text{IL}-1\beta]}\right)\text{Output}}{\left(\frac{[[\text{IL}-1\text{ra}]}{[\text{IL}-1\beta]}\right)\text{Input}}$$

$$\text{Equation 3: Anti-IL-1 Efficacy Ratio} = \frac{\left(\frac{[\text{sIL}-1\text{RII}]}{[\text{IL}-1\alpha]}\right)\text{Output}}{\left(\frac{[\text{sIL}-1\text{RII}]}{[\text{IL}-1\alpha]}\right)\text{Input}}$$

In some embodiments, the concentration of more than one inflammatory cytokine and/or the concentration of more than one anti-inflammatory cytokine can be combined to calculate an Efficacy Ratio, as long as the anti-chosen inflammatory cytokines mediate the activity of the chosen inflammatory cytokines. In a Combined Efficacy Ratio, the inflammatory cytokine comprises a plurality of inflammatory cytokines, and/or the anti-inflammatory cytokine comprises a plurality of anti-inflammatory cytokines. The Combined Efficacy Ratio is shown in Equation 4.

$$\text{Equation 4: Combined Efficacy Ratio} = \frac{\left(\frac{[\text{sum of anti}-\text{inflammatory cytokines}]}{[\text{sum of inflammatory cytokines}]}\right)\text{Output}}{\left(\frac{[\text{sum of anti}-\text{inflammatory cytokines}]}{[\text{sum of inflammatory cytokines}]}\right)\text{Input}}$$

For example, if an inflammatory disorder is associated with increased levels of IL-1$\alpha$ and IL-1$\beta$, then the sum of the IL-1$\alpha$ and IL-1$\beta$ concentrations can be used to determine the Anti-IL-1 Efficacy Ratio. Likewise, the anti-inflammatory cytokine can be a sum of the concentrations of IL-1ra and IL-1RII. When anti-inflammatory cytokines and/or inflammatory cytokines are combined, a Combined Efficacy Ratio of at least about 1 indicates that the composition is suitable for treating an inflammatory disorder. A Combined Efficacy Ratio of greater than or equal to 1 also indicates that the composition is suitable for treating an inflammatory disorder. Example Combined Efficacy Ratios are shown in Equations 5, 6, and 7.

$$\text{Equation 5: Combined Anti-IL-1 Efficacy Ratio} = \frac{\left(\frac{[\text{IL}-1\text{ra}]+[\text{sIL}-1\text{RII}]}{[IL-1\alpha]+[\text{IL}-1\beta]}\right)\text{Output}}{\left(\frac{[[\text{IL}-1\text{ra}]+[sIL-1RII]}{[IL-1\alpha]+[\text{IL}-1\beta]}\right)\text{Input}}$$

$$\text{Equation 6: Combined Anti-IL-1 Efficacy Ratio} = \frac{\left(\frac{[\text{IL}-1\text{ra}]+[\text{sIL}-1\text{RII}]}{[\text{IL}-1\beta]}\right)\text{Output}}{\left(\frac{[\text{IL}-1\text{ra}]+[\text{sIL}-1\text{RII}]}{[\text{IL}-1\beta]}\right)\text{Input}}$$

$$\text{Equation 7: Combined TNF}\alpha \text{ Efficacy Ratio} = \frac{\left(\frac{[\text{sTNF-RI}]+[\text{sTNF-RII}]}{[\text{TNF}\alpha]}\right)\text{Output}}{\left(\frac{[\text{sTNF-RI}]+[\text{sTNF-RII}]}{[\text{TNF}\alpha]}\right)\text{Input}}$$

In some embodiments, the Efficacy Ratio can be modified to analyze whether processing a biological material in a device results in an improvement of two or more signaling cascades. A Multiple Pathway Efficacy Ratio is shown in Equation 8.

$$\text{Equation 8: Multiple Pathway Efficacy Ratio}$$

$$= \frac{\left(\frac{[\text{sum of first anti-inflammatory cytokines}]}{[\text{sum of first inflammatory cytokines}]}\right)\text{Output}}{\left(\frac{[\text{sum of first anti-inflammatory cytokines}]}{[\text{sum of first inflammatory cytokines}]}\right)\text{Input}} \text{ and } \frac{\left(\frac{[\text{sum of second anti-inflammatory cytokines}]}{[\text{sum of second inflammatory cytokine}]}\right)\text{Output}}{\left(\frac{[\text{sum of second anti-inflammatory cytokines}]}{[\text{sum of second inflammatory cytokine}]}\right)\text{Input}}$$

In Equation 8, when both ratios are equal to or greater than 1, an improvement in both signaling cascades in the composition is indicated. Such a composition could be used to treat an inflammatory disorder associated with the first inflammatory cytokine, an inflammatory disorder associated with the second inflammatory cytokine, or an inflammatory disorder associated with both the first and second inflammatory cytokines. Again, it is important that in both ratios the inflammatory cytokines mediate the activity of the corresponding inflammatory cytokines. Equation 9 is an example Multiple Pathway Efficacy Ratio, wherein the inflammatory cytokines are IL-1$\beta$ and TNF$\alpha$.

$$\text{Equation 9: Multiple Pathway Efficacy Ratio}$$

$$= \frac{\left(\frac{[\text{IL-1ra}]+[\text{sIL-1RII}]}{[\text{IL-1}\beta]}\right)\text{Output}}{\left(\frac{[\text{IL-1ra}]+[\text{sIL-1RII}]}{[\text{IL-1}\beta]}\right)\text{Input}} \text{ and } \frac{\left(\frac{[\text{sTNF-RI}]+[\text{sTNF-RII}]}{[\text{TNF}\alpha]}\right)\text{Output}}{\left(\frac{[\text{sTNF-RI}]+[\text{sTNF-RII}]}{[\text{TNF}\alpha]}\right)\text{Input}}$$

In yet other embodiment, the Efficacy Ratio can be modified to analyze whether processing a biological material in a device results in an improvement of one of two or more inflammatory signaling cascades. A Combined Pathway Efficacy Ratio, which indicates whether a device results in an improvement of one of two or more inflammatory signaling cascades is shown in Equation 10.

$$\text{Equation 10: Combined Pathway Efficacy Ratio}$$

$$= \frac{\left(\frac{[\text{sum of first anti-inflammatory cytokines}]}{[\text{sum of first inflammatory cytokines}]}\right)\text{Output}}{\left(\frac{[\text{sum of first anti-inflammatory cytokines}]}{[\text{sum of first inflammatory cytokines}]}\right)\text{Input}} \text{ or } \frac{\left(\frac{[\text{sum of second anti-inflammatory cytokines}]}{[\text{sum of second inflammatory cytokine}]}\right)\text{Output}}{\left(\frac{[\text{sum of second anti-inflammatory cytokines}]}{[\text{sum of second inflammatory cytokine}]}\right)\text{Input}}$$

In Equation 10, when one of the ratios corresponding to the first or second anti-inflammatory cytokine is equal to or greater than 1, then an improvement in that signaling cascade is indicated. When the ratios corresponding to both the first anti-inflammatory cytokine and the second anti-inflammatory cytokine are both greater than or equal to 1, then Equation 10 is essentially the same as Equation 8. However, Equation 10 is useful, for example, when an inflammatory disorder is associated with two or more inflammatory cytokines, and a medical professional desires to generate an anti-inflammatory composition that will mediate the activity of at least one of the inflammatory cytokines. Equation 11 is an example Combined Pathway Efficacy Ratio, wherein the activity of either IL-1$\beta$ or TNF$\alpha$ is to be mediated.

$$\text{Equation 11: Combined Pathway Efficacy Ratio}$$

$$= \frac{\left(\frac{[\text{IL-1ra}]+[\text{sIL-1RII}]}{[\text{IL-1}\beta]}\right)\text{Output}}{\left(\frac{[\text{IL-1ra}]+[\text{sIL-1RII}]}{[\text{IL-1}\beta]}\right)\text{Input}} \text{ or } \frac{\left(\frac{[\text{sTNF-RI}]+[\text{sTNF-RII}]}{[\text{TNF}\alpha]}\right)\text{Output}}{\left(\frac{[\text{sTNF-RI}]+[\text{sTNF-RII}]}{[\text{TNF}\alpha]}\right)\text{Input}}$$

Methods for Using Efficacy Ratios

As discussed above, the present technology provides various methods for the design, manufacture, control, use and other management of protein solutions and devices for making protein solutions, using Efficacy Ratios. In general, such methods comprise the determination of one or more Efficacy Ratios and, in various embodiments, performance of further steps depending on the value of an Efficacy Ratio. In various embodiments, the Efficacy Ratio may be determined consistent with the desired composition and utility of anti-inflammatory compositions that are suitable to treat a specific disorder associated with an inflammatory cytokine.

In various embodiments, an Efficacy Ratio may be compared with another Efficacy Ratio or a predetermined value, and further steps may be performed based on the comparison. For example, an anti-inflammatory composition may be considered to be suitable for treating an inflammatory disorder if an Efficacy Ratio determined in a method of this technology is at least about 1 (i.e., about 1 or greater than 1) or is greater than or equal to 1, in order for an anti-inflammatory composition to be. As discussed further below, in some embodiments, an anti-inflammatory composition generated for treating an inflammatory disorder associated with elevated levels of IL-1β should have an Anti-IL-1 Efficacy Ratio of 1 or greater.

In some embodiments, further steps performed based on a comparison of an Efficacy Ratio include preparing an anti-inflammatory composition for administration to a human or other animal subject. For example, when the Efficacy Ratio is at least about 1, the anti-inflammatory composition can be prepared for effecting delivery. If the anti-inflammatory composition has an Efficacy Ratio of less than 1, then it can be re-processed, used for a different purpose, or discarded.

Such "preparing" may comprise transferring the anti-inflammatory composition into a delivery device, such as a syringe, or into a storage container if both the first Efficacy Ratio and the second Efficacy Ratio are 1 or greater. In some embodiments, the determining of the input and output concentrations of the anti-inflammatory cytokine and inflammatory cytokines may be performed by obtaining a sample of the anti- inflammatory cytokine composition from the vessel in which the composition is made, such as the production devices for making protein-rich solutions using solid extraction materials described above. For example, the composition may be drawn from the production device using a syringe or other delivery device suitable for administering the composition to a subject.

A sample of the composition may be injected into a diagnostic device (such as described below) for determining of the output concentrations of the anti-inflammatory cytokine and inflammatory cytokines.

Although not part of the claimed method, administering of an anti-inflammatory composition may be performed in a "point-of-care" method for treating a human or other animal subject with the anti-inflammatory composition. As referred to herein, a "point-of-care" method is performed at a time proximate to the administration of the anti-inflammatory composition to the subject being treated. Such methods may be performed at a location proximate, such as in the same room (for example, bed side) or otherwise immediately adjacent, to the mammalian subject to be treated. Thus, in some embodiments, one or more steps of methods resulting in the calculation of an Efficacy Ratio are performed proximate to preparing the anti-inflammatory composition for administration. For example, the steps of obtaining a biologic material, determining the input concentrations, processing of the biological material, determining output concentrations, and calculating an Efficacy Ratio are performed proximate to administration of the anti- inflammatory composition to the subject.

As exemplified in Fig. 6, the current technology provides a method 600 for generating an anti-inflammatory composition. As shown in block 605, the method 600 comprises obtaining a biological material. The biological material can be any biological material that generates anti-inflammatory cytokines. For example, the biological material can be selected from the group consisting of whole blood, a blood fraction, adipose tissue, adipocytes, bone marrow aspirate, concentrated bone marrow aspirate, synovial fluid, urine, and mixtures thereof. In some embodiments, the biological material is a plurality of cytokine producing cells.

Obtaining a biological material includes either obtaining a biological material from storage, or drawing or otherwise removing the biological material from a subject. In various embodiments, the anti-inflammatory cytokine composition is autologous to a subject in need of treatment for an inflammatory disorder. Therefore, the biological material is obtained from the subject in need of treatment for the inflammatory disorder. Alternatively, the biological material could be obtained from storage, wherein the biological material was previously drawn from the subject in anticipation of treatment. In other embodiments, the anti-inflammatory cytokine composition is allogeneic to a subject in need of treatment for an inflammatory disorder. In such embodiments, the biological material can be obtained from another subject at the time of performing the method, or the biological material can be obtained from storage. As used herein, a "subject" can be a human or non-human mammal.

In block 610, the method 600 comprises determining input cytokine concentrations. As described above, determining input cytokine concentrations includes determining input concentrations of an anti-inflammatory cytokine and of an inflammatory cytokine in the biological material, wherein the anti-inflammatory cytokine inhibits the activity of the inflammatory cytokine.

In block 615, the method 600 comprises processing the biological material to form an anti-inflammatory composition. In various embodiments, processing can be performed with a device for generating an anti-inflammatory composition to form an anti-inflammatory composition. Processing the biological material in a device can be performed by any method known in the art, including one or more methods discussed above. Thus, for example, whole blood may be processed in a centrifuge to generate a fraction of concentrated red blood cells, a platelet-rich fraction, and a platelet-poor fraction. As further described above, processing may include contacting the biological material with a solid extraction material.

In block 620, the method 600 comprises determining output cytokine concentrations. Determining output cytokine concentrations includes determining output concentrations of the anti- inflammatory cytokine and of the inflammatory cytokine in the anti- inflammatory composition, wherein the cytokines are the same cytokines examined in block 610.

As described above, determining the input concentrations of inflammatory cytokines and anti-inflammatory cytokines in block 610 and the output concentrations of inflammatory cytokines and anti-inflammatory cytokines in block 620 can be performed by any method known in the art. Non-limiting examples of methods for determining the concentration of the cytokines include the diagnostic methods discussed above.

In block 625, the method 600 comprises calculating an Efficacy Ratio. Calculating an Efficacy Ratio can be performed by using any of Equations 1, 4, 8, and 10 described above.

In block 630, the method 600 comprises keeping or discarding the anti-inflammatory composition. In some embodiments, the anti-inflammatory composition is kept if the Efficacy Ratio is greater than or equal to 1. Keeping the anti-inflammatory composition may comprise preparing the anti-inflammatory composition for administration, as described above, such as by transferring the anti-inflammatory composition into a syringe or other delivery device for effecting delivery or into a storage container. Alternatively, the anti-inflammatory composition can be discarded, reprocessed, or repurposed when the Efficacy Ratio is less than 1.

As described above, determining input concentrations of an anti-inflammatory cytokine and of an inflammatory cytokine in the biological material in block 610 and output concentrations of the inflammatory cytokine and inflammatory cytokine in the anti-inflammatory composition in block 620 can be performed by methods and devices for measuring cytokine levels in fluids among those known in the art.

In various embodiments, the steps of blocks 610, 615, 620, and, optionally, 625 are performed by a diagnostic device of the present technology. The diagnostic device comprises an inlet port operable to intake a sample of a biological material and a sample of the anti-inflammatory composition, a detection chamber, in fluid communication with the inlet port, operable to detect, and a display, in communication with the detection chamber. The detection chamber is operable to detect the concentration of a first anti-inflammatory cytokine in the biological sample ([first anti-inflammatory cytokine] sample), and the concentration of a first inflammatory cytokine in the biological material ([first inflammatory cytokine] sample); and the concentration of the first anti-inflammatory cytokine in the anti-inflammatory composition ([first anti-inflammatory cytokine] composition) and the concentration of the first inflammatory cytokine in the anti-inflammatory composition ([first inflammatory cytokine] composition), in the anti-inflammatory composition. The diagnostic device measures concentrations of specific cytokines, inflammatory and anti-inflammatory, from the biological material input and the anti-inflammatory composition output, by changes in electrical signals or by signals generated after cytokines bind to antibodies. The display is operable to display an indicia of the concentrations of the first anti-inflammatory cytokine in the biological sample, the first inflammatory cytokine in the biological material, the first anti-inflammatory cytokine in the anti-inflammatory composition, and the first inflammatory cytokine in the anti-inflammatory composition.

The diagnostic device automatically calculates an Efficacy Ratio according to Equation 1. The calculated Efficacy Ratio can be displayed on the display by any indicia suitable to convey the information regarding the Efficacy Ratio and the recommended action in block 630 (i.e., to prepare the composition for administration or otherwise "keep" the composition, or to discard the composition (i.e., to discard, further process or repurpose the composition). For example, the indicia may be a number, a bar chart, or a symbol, such as a "+" when the ratio is equal to or greater than 1, or a "-" when the ratio is less than 1. The diagnostic device can be selected from a plurality of devices, which are each individually calibrated to measure a specific anti-inflammatory-inflammatory cytokine pair, wherein the anti-inflammatory cytokine mediates the activity of the inflammatory cytokine. Alternatively, at least one anti-inflammatory-inflammatory cytokine pair (as discussed above) can be selected for measurement on a device, which is capable of measuring a plurality of cytokine pairs. The diagnostic device can be used to determine Efficacy Ratios at a time prior to when the anti-inflammatory composition will be administered, or the device can be used to determine an Efficacy Ratio in a point-of-care method, as described above. Thus, the diagnostic may be used at a time proximate to the administration of the anti-inflammatory composition to the subject being treated. Such a diagnostic device that calculates Efficacy Ratios at a point-of-care is referred to as a "point-of-care diagnostic."

In some embodiments, input and output cytokine concentrations can be determined, for example, by ELISA analysis, and the concentrations can be copied into a spreadsheet or calculating device, either of which is programmed to automatically calculate Efficacy Ratios. In this embodiment, the spreadsheet or calculator calculating device is programmed, developed, or manufactured for the purpose of calculating Efficacy Ratios.

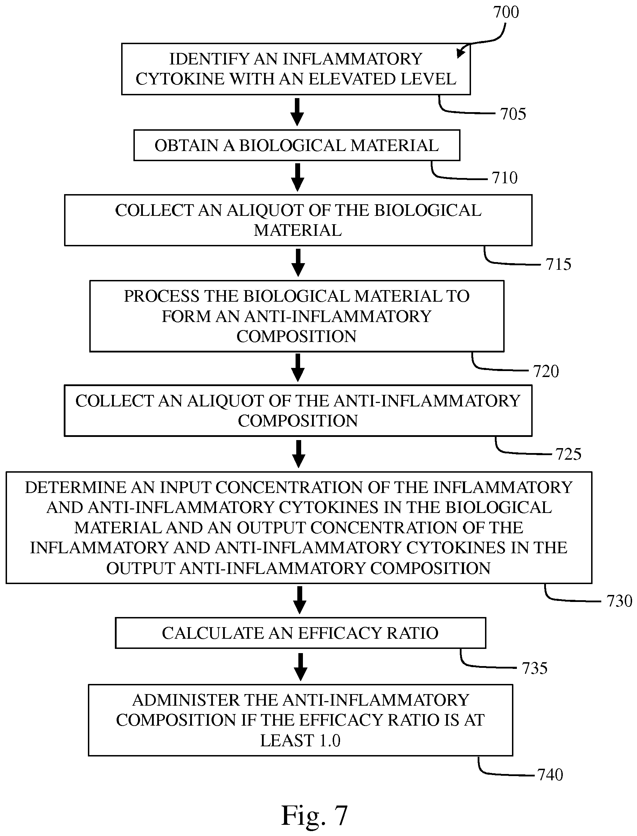

Although not part of the claimed method, as shown in Fig. 7, anti-inflammatory compositions may be used in a method 700 for treating an inflammatory disorder in a human or non-human mammal patient. The inflammatory disorder can be any disorder that is associated with elevated levels of an inflammatory cytokine. The inflammatory disorder may be rheumatoid arthritis, osteoarthritis, osteolytis, tendonitis, synovitis, peripheral vascular disease, or inflammatory respiratory diseases (such as chronic obstructive pulmonary disease, fibrosis, emphysema, acute respiratory distress syndrome, and pneumonia).

In block 705, the method 700 comprises identifying an inflammatory cytokine that has an elevated level associated with the inflammatory disorder in the patient. As used herein, an "elevated level" can be an elevated concentration or activity. Identifying can be performed by obtaining a biopsy from the patient and screening the biopsy for a cytokine with an elevated level. Alternatively many inflammatory disorders are well characterized in the art, including knowledge of inflammatory cytokines associated with the disorders. Therefore, a medical professional, such as a physician, physician's assistant, nurse, or other medical technician, may deduce from the disorder itself what inflammatory cytokine is elevated.

In block 710, the method 700 comprises obtaining a biological material. The biological material can be any material, tissue, or plurality of cells that produces cytokines. The biological material may be obtained from the patient in order to generate an autologous anti-inflammatory composition. The biological material can be drawn from the patient at the time of performing the method 700, or the biological material could be obtained from storage, having been drawn previously from the patient in anticipation of treatment. The biological material may be allogeneic to the patient. As shown in block 715, the method 700 includes collecting an aliquot of the biological material.

In block 720, the method 700 comprises processing the biological material. Processing may include contacting the biological material with a solid extraction material. Non-limiting examples of solid extraction materials include corundum, quartz, titanium, dextran, agarose, polyacrylamide, polystyrene, polyethylene, polyvinyl chloride, polypropylene, and combinations thereof. The solid extraction material may be polyacrylamide. The biological material may be processed in a device that contains the solid extraction material. However, it is understood that any form of processing described herein can be performed. Processing the biological material results in an anti- inflammatory cytokine composition.

In block 725, the method 700 comprises collecting an aliquot of the anti-inflammatory composition. In block 730, the method 700 comprises determining an input concentration of the inflammatory cytokine of an anti-inflammatory cytokine that mediates the activity of the inflammatory cytokine from the aliquot collected in block 715, and determining output concentrations of the same cytokines in the aliquot collected in block 725. The concentrations are then used for calculating an Efficacy Ratio in block 735. As described above, blocks 730 and 735 can be performed with a point-of-care diagnostic device configured to calculate Efficacy Ratios. In block 740, the method 700 comprises administering the anti-inflammatory composition to the patient at or near a site of inflammation if the Efficacy Ratio is at least 1. As discussed above, administering the anti-inflammatory composition may include transferring the anti-inflammatory composition to a device for effecting delivery, such as a syringe or other delivery device, or administering the anti-inflammatory composition directly from a device in which the biological material was processed. If the Efficacy Ratio is less than 1, then the anti-inflammatory composition may not be administered to the patient. When the Efficacy Ratio is less than 1, the anti-inflammatory composition can be re-processed, used for a different purpose, or discarded.

The present technology also provides kits for the treatment of an inflammatory disorder. Such kits may comprise a device for the processing of the biological material, and a diagnostic device. The device for the processing of the biological material can be any device described herein, and is adapted for use in processing a biological material to produce an anti-inflammatory composition in a method of this technology. The diagnostic device provided in the kit can be a device or one or more components of a device for measuring cytokine concentrations, which is operable to determine the concentration of one or more anti-inflammatory cytokines, one or more inflammatory cytokines, or both in the anti-inflammatory composition or the biological material from which the composition is made using the device. Non-limiting examples of such diagnostics include those discussed above. In some embodiments, the diagnostic device is a diagnostic device as described above.

The present technology further comprises a method for determining whether a device is suitable for generating an anti-inflammatory composition from a biological material. The method comprises obtaining a biological material, determining input concentrations of an inflammatory cytokine and of an anti-inflammatory cytokine that mediates the activity of the inflammatory cytokine in the biological material, processing the biological material with the device to generate an anti-inflammatory composition, determining output concentrations of the inflammatory and anti-inflammatory cytokines in the anti-inflammatory composition, and calculating an Efficacy Ratio. Finally, if the Efficacy Ratio is equal to or greater than 1, then the device is suitable for generating an anti-inflammatory composition. If the Efficacy Ratio is less than 1, then the device may not be suitable for generating an anti-inflammatory composition.

Additionally, the present technology provides devices for generating an anti-inflammatory composition from a biological material, comprising a device for processing an anti-inflammatory composition as discussed above and one or more components of a diagnostic device as discussed above. For example, the device may comprise an inlet port in fluid communication with a mixing chamber containing a solid extraction material, and a diagnostic device in fluid communication with the input port, the mixing chamber, or both, wherein the device is operable to determine one or both of the input and ouput concentrations of inflammatory and anti-inflammatory cytokines, as in method of the present technology. In some embodiments, the device is operable to determine both the input and output concentrations. The device may be operable to display an indicia of an Efficacy Ratio, as discussed above, such as to indicate the whether an anti-inflammatory composition has been produced with an Efficacy Ratio greater than or equal to a desired level (e.g., 1) when a biological material contacts the solid extraction material. The device may display the indicia as a real time indication of the Efficacy Ratio, allowing control of the duration of the processing step of the present method, e.g., control of the period of time during which the biological material is contacted with polyacrylamide beads or other solid extraction material.

The present technology further provides a method for assessing the efficacy of an anti-inflammatory composition. The method comprises obtaining a biological material comprising a first inflammatory cytokine and a first anti-inflammatory cytokine from a human or other animal subject; determining the input concentration of the first anti-inflammatory cytokine ([first anti-inflammatory cytokine] input), and the input concentration of the first inflammatory cytokine ([first inflammatory cytokine] input), in the biological material; processing the biological material to produce an anti-inflammatory composition having an increased concentration of the first anti-inflammatory cytokine; determining output concentration of the first anti-inflammatory cytokine ([first anti-inflammatory cytokine] output) and the output concentration of the first inflammatory cytokine ([first inflammatory cytokine] output), in the anti-inflammatory composition; and calculating a first Efficacy Ratio pursuant to Equation 1. The steps of such methods may include one or more of the steps discussed above regarding methods for generating anti-inflammatory compositions.

The present technology also provides methods for comparing the efficacy of a first device and a second device for making anti-inflammatory compositions having increased concentrations of anti-inflammatory cytokines from biological materials. The method comprises performing a trial method for generating an anti-inflammatory composition performed using the first device, and obtaining an Efficacy Ratio for the first device; performing the trial method using the second device, and obtaining an Efficacy Ratio for the second device; and comparing the Efficacy Ratio for the first device to the Efficacy Ratio for the second device, wherein the Efficacy Ratios are calculated according to Equation 1. Performing a trial method comprises obtaining a biological material comprising a first inflammatory cytokine and a first anti-inflammatory cytokine from a human or other animal subject; determining the input concentration of the first anti-inflammatory cytokine ([first anti-inflammatory cytokine] input), and the input concentration of the first inflammatory cytokine ([first inflammatory cytokine] input), in the biological material; processing the biological material in the first or second device to produce an anti-inflammatory composition having an increased concentration of the first anti-inflammatory cytokine; determining output concentration of the first anti-inflammatory cytokine ([first anti-inflammatory cytokine] output) and the output concentration of the first inflammatory cytokine ([first inflammatory cytokine] output), in the anti-inflammatory composition; and calculating a first Efficacy Ratio pursuant to Equation 1. The device that results in an anti-inflammatory composition with a the higher Efficacy Ratio has a higher efficacy for generating the anti-inflammatory composition. The steps of such methods may include one or more of the steps discussed above regarding methods for generating anti-inflammatory compositions. For example, i_various embodiments, one or both of the first and second devices comprises a solid extraction material.

Embodiments of the present technology are further illustrated through the following non-limiting examples.

Example 1

Preparing and Characterizing an Anti-inflammatory Composition

An anti-inflammatory protein solution composition rich in interleukin-I receptor antagonist is prepared from seven consented human providers. Blood (55 mL) is drawn into a 60 cc syringe with 5 mL of anticoagulant citrate dextrose solution A (ACD-A, Citra Labs, Braintree, MA). Platelet-rich plasma (PRP) is created using the GPS® III platelet concentration system (800-1 003A, Biomet Biologics, Warsaw, Indiana) according to the instructions for use. The solution is generated by adding 6 mL of PRP to a modified Plasmax™ device containing 1 gram of polyacrylamide beads (Biomet Biologics, Warsaw, IN). The IL-Ira solution is removed from the Plasmax™ devices and frozen at minus 50°C for the assay. Cytokine content is assayed on a 16-plex ELISA (Searchlight Protein Array, Aushon Biosystems, Billerica, MA). The analytes included IL-4, IL-10, IL-11, IL-I3, IL-Ira, IFN-$\gamma$, sTNF-RI, sTNF-RII, IL-1$\alpha$, IL-1$\beta$, TNF-$\alpha$, IL-17, IL-18, bFGF, TBF-$\beta$1, and TBF-$\beta$2.

The solution contains both anabolic (bFGF, TGF-$\beta$1, TGF-$\beta$2 (see Table 3)) and anti-inflammatory (IL-1ra, sTNF-RI, sTNF-RII, IL-4, IL-10, IL-11, IL-13, IFN-$\gamma$, (see Table 4)) cytokines without expressing large doses of catabolic cytokines (IL-1$\alpha$, IL-1$\beta$, TNF-$\alpha$, IL-17, IL-18 (see Table 5)). The anti-inflammatory cytokines IL-Ira and sTNF-R are all detected in ng/mL quantities, while all of the catabolic analytes were in pg/mL quantities. However, donor-to-donor variability is detected. Correlations between the catabolic cytokines IL-1 and TNF-a and anti-inflammatory analytes IL-1ra and sTNF-R are compared, but no large correlations detected (Table 6). On average, there is about 13,260 times more IL-Ira than IL-1$\alpha$ and about 7,561 times more than IL-1$\beta$.

Table 3. Anabolic cytokines in the solution.

| Donor | bFGF | TGF-β1 | TGF-β2 |
|---|---|---|---|
| 1 | 18.5 | 1,458,008 | 153,833 |
| 2 | 10.7 | 1,137,404 | 119,545 |
| 3 | 11.9 | 585,298 | 70,544 |
| 4 | 4.9 | 1,342,442 | 162,707 |
| 5 | 20.0 | 1,579,361 | 204,670 |
| 6 | 7.7 | 1,393,746 | 170,345 |
| 7 | 13.9 | 1,474,155 | 174,502 |
| Average | 12.5 | 1,281,488 | 150,878 |
| ± SD | ± 5.5 | ± 336,345 | ± 43,617 |

Table 4. Anti-inflammatory cytokines in the solution.

| Donor | IFN-γ | IL-4 | IL-10 | IL-13 | IL-1ra | TNF-RI | TNF-RII | IL-11 |
|---|---|---|---|---|---|---|---|---|
| 1 | <0.4 | 2.1 | 0.5 | 3. 5 | 9,660 | 2,728 | 2,249 | <2.0 |
| 2 | <0.4 | 1.3 | 0.3 | 2.8 | 17,477 | 5,120 | 2,900 | <2.0 |
| 3 | <0.4 | <0.8 | 0.3 | 0.1 | 23,126 | 6,247 | 2,446 | <2.0 |
| 4 | 40.4 | 59.9 | 8.9 | 19.9 | 10,458 | 4,374 | 2,612 | <2.0 |
| 5 | 30.2 | 33.9 | 23.3 | 15.8 | 13,462 | 2,763 | 1,394 | <2.0 |
| 6 | 2.6 | 23.3 | 1.4 | 25.6 | 8,813 | 2,992 | 2,716 | <2.0 |
| 7 | 0.7 | 1.2 | 0.6 | 1.8 | 11,277 | 3,330 | 1,915 | <2.0 |
| Average | 10.7 | 17.5 | 5.0 | 9.9 | 13,468 | 3,936 | 2,319 | <2.0 |
| ± SD | ± 17.0 | ±22.9 | ± 8.7 | ±10.3 | ± 5,154 | ± 1,356 | ± 520 | ± 0 |

Table 5. Catabolic cytokines in the solution.

| Donor | IL-17 | TNF-α | IL-1α | IL-1β | IL-18 |
|---|---|---|---|---|---|
| 1 | 3.1 | 16.0 | <0.8 | 1.5 | 239 |
| 2 | 1.2 | <2.3 | 2.5 | 3.3 | 559 |
| 3 | 0.7 | <2.3 | 1.8 | 2.3 | 511 |
| 4 | 28.9 | 195 | 0.8 | 1.3 | 329 |
| 5 | 33.8 | 661 | 0.8 | 2.0 | 450 |
| 6 | 22.0 | 105 | 0.3 | 1.7 | 333 |
| 7 | 6.7 | <2.3 | 1.9 | 1.0 | 787 |
| Average | 13.8 | 141 | 1.3 | 1.9 | 458 |
| ± SD | ±14.1 | ±241 | ± 0.8 | ± 0.8 | ± 183 |

Table 6. Correlation analysis.

| Analytes compared | $R^2$ | Ratio |
|---|---|---|
| IL-1ra and IL-1α | 0.46 | 13,260X |
| IL-1ra and IL-1β | 0.45 | 7,561X |
| TNF-RI and TNF-α | 0.17 | 945X |
| TNF-RII and TNF-α | 0.47 | 477X |

Example 2

Generation of IL-1ra from Platelet-Rich Plasma.

An IL-1ra-rich solution is created as follows. Whole blood (70 mL) anticoagulated (10%) with ACD-A (Braintree, Massachusetts, USA) is drawn from 5 healthy volunteers. A portion (10 mL) is reserved for a whole blood measurement. Platelet-rich plasma (PRP) (6 mL) is produced using the GPS® II System (Biomet Biologics, LLC, Warsaw, Indiana, USA). Complete blood counts are collected for the whole blood and PRP samples following a validated procedure, as described in Woodell-May JE, Ridderman DN, Swift MJ, Higgins J. "Producing Accurate Platelet Counts for Platelet Rich Plasma: Validation of a Hematology Analyzer and Preparation Techniques for Counting" J. Craniofac. Surg. (2005) Sep. 16(5):749-56.

Following the PRP production, 5 mL of the PRP is added to a modified plasma concentration device (Plasmax™, Biomet Biologics LLC, Warsaw, Indiana, USA) and incubated with polyacrylamide desiccating beads in the device for 24 hours at room temperature. Following the contact with polyacrylamide beads the electromagnetic field, the plasma concentration device is centrifuged to separate the serum fraction.

To analyze baseline IL-1ra levels at time zero, the whole blood and PRP samples are activated with 50 $\mu$L of thrombin and 10 % CaC12 (1,000 units/mL). A blood clot is formed and incubated for 30 minutes at room temperature. Following incubation, the clot is centrifuged for 5 minutes at 3,000 rpm. Serum is collected from the clots and retained for ELISA analysis. The serum fraction from the plasma concentrator does not require activation by thrombin, and is tested directly. All samples are analyzed for IL-1ra using an ELISA kit (IL-1ra Quantikine™ Kit, R&D Systems, Minneapolis, Minnesota, USA).

The PRP samples result in about an eight-fold increase in platelets, about five-fold increase in total white blood cells (WBCs), about nine-fold increase in the monocyte fraction of the WBCs, and about a three-fold increase in the PMN fraction of the WBCs. The IL-1ra production in the whole blood and PRP samples is correlated most closely to the WBC concentration. The five-fold increase in the PRP is likely due to the increase in WBCs, and both the whole blood and PRP IL-1ra values can be considered baseline IL-1ra content. This is in contrast to the 195-fold increase in IL-1ra following incubation in the plasma concentrator. This plasma concentration device typically results in a 3-fold increase in plasma protein concentration due to a volume reduction caused by the desiccation process. This 3-fold decrease in volume does not account for the levels of increase seen in the amount of IL-1ra. Therefore, this level of increase indicates stimulation of WBCs to produce IL-1ra during the contact with the solid extraction material (e.g., polyacrylamide beads) and electromagnetic field stimulation.

Correlation analysis demonstrates that IL-1ra production is more closely correlated with the increase in WBCs than the platelet content. The IL-1ra levels do not correlate as closely with the WBC population in the PRP. This is not surprising since the WBC are not activated, and the serum is collected by thrombin activation of the plasma. However, it is possible that the WBC, once activated in the plasma concentration device, participate in the significant production of IL-1ra seen in this example.

Example 3

Production of Protein Solution from PRP

Anticoagulated blood (120 cc) is collected from 5 human donors. Platelet-rich plasma (PRP) is prepared using GPS® III disposables (Biomet Biologics LLC, Warsaw, Indiana, USA). PRP is loaded into modified plasma concentration devices (Plasmax™, Biomet Biologics LLC, Warsaw, Indiana, USA) and processed. The output is divided into 4 groups: IL-1ra in concentrated plasma with and without thrombin activation (1000 U/mL in 1M CaCl2), or cell-free IL-1ra with and without thrombin activation. IL-1ra is measured using ELISA (R&D Systems) over time.

The PRP contacts polyacrylamide beads in the Plasmax™ device while electromagnetic field stimulation is provided using a capacitively coupled electromagnetic field.

Unclotted PRP produces an average of about 50 ng over 24 hrs. The cell-free samples produce about 34 ng without changing over 24 hrs. Once clotted, the elution of IL-1ra is slowed, with only about 30% being eluted after 10 hours. Release in the cell-free samples is also delayed, but eluted 100% of available IL-1ra after 10 hours.

Example 4

Generation of Protein Solution and Characterization of Cytokine Levels In Healthy Subjects and Osteoarthritis Subjects

An Autologous Protein Solution (APS) from healthy patients are prepared as follows for the measurement of growth factors. 72 ml of anticoagulated whole blood are drawn by venipuncture from each of six donors. 3 ml of each donor's anticoagulated whole blood are aliquoted into microcentrifuge tubes and frozen at -50°C. 60 ml of the anticoagulated whole blood is loaded into GPS® III disposable devices (Biomet Biologics LLC, Warsaw, Indiana, USA), which is processed according to the manufacturer's instructions to produce PRP. The PRP is removed from the GPS® III devices and added to Plasmax™ devices (Biomet Biologics LLC, Warsaw, Indiana, USA), which is processed according to the manufacturer's instructions to produce APS. APS is extracted from each device, aliquoted into microcentrifuge tubes, and frozen at - 50°C. Each sample, whole blood and PRP, is subjected to three freeze-thaw cycles. Quantikine Human Immunoassays (R&D Systems, Inc., Minneapolis, MN) for VEGF, PDGF-BB, PDGF-AB, EGF, TGF-β1, TGF-β2, and IGF-1 are run in duplicate according to the manufacturer's instructions for each APS and whole blood sample.

APS from healthy patients is prepared as above for the measurement of anti-inflammatory cytokines. Quantikine Human Immunoassays (R&D Systems, Inc., Minneapolis, MN) for IL-1ra, IL-1β, IL-8, sTNF-RI, TNF-α, IL-6, sTNF-RII, IL-10, IL-13, and IL-4 are run in duplicate according to the manufacturer's instructions for each APS and whole blood sample. Immunoassays are also performed to detect hepatocyte growth factor (HGF) and soluble IL-1RII.

APS from 105 osteoarthritis patients is prepared as above for the measurement of growth factors anti-inflammatory cytokines. The APS is stored at -50°C or in dry ice.

Cytokine concentrations are compared between healthy donors and OA patients in baseline blood and APS. IL-1β is concentrated at a higher level in OA patients, but the fold increase is still much lower than that of IL-1ra. Other cytokines and growth factors that are concentrated at least to the level of that observed in healthy donors include sTNF-RI, IGF-I, IL-8, VEGF, and IL-6. The soluble cytokines sTNF-RII and sIL-1RII are concentrated to a level not quite as high but very similar to the healthy concentration level. The results are displayed in Table 7.

Table 7. Concentration of growth factors and anti-inflammatory cytokines from APS derived from healthy patients and patients with osteoarthritis (in pg/ml).

| Cytokine | | Baseline | | APS | | Fold Increase |
|---|---|---|---|---|---|---|
| | | Average | StDev | Average | StDev | Average |
| VEGF | Healthy | 276 | 109 | 742 | 494 | 2.7 |
| | OA | 484 | 201 | 1710 | 1025 | 3.8 |
| IL-1β | Healthy | 3.4 | 2 | 3.8 | 0.8 | 1.1 |
| | OA | 3.3 | 1.1 | 8.9 | 7.3 | 2.8 |
| IL-8 | Healthy | 74 | 16 | 315 | 198 | 4.3 |
| | OA | 73.5 | 29.6 | 287.9 | 192.7 | 4.2 |
| IL-6 | Healthy | 3.1 | 0.4 | 3.4 | 0.7 | 1.1 |
| | OA | 1.8 | 1.3 | 3 | 3.5 | 1.6 |
| TNF-α | Healthy | ND | ND | 3.4 | 0.7 | ND |
| | OA | 2.4 | 2 | 4.3 | 3 | 5.3 |
| IL-1ra | Healthy | 8092 | 2536 | 30853 | 16737 | 3.8 |
| | OA | 7576 | 2469 | 41896 | 19669 | 5.9 |
| sTNF-RII | Healthy | 2485 | 338 | 9491 | 1387 | 3.8 |
| | OA | 1491 | 492 | 5060 | 1946 | 3.5 |
| PDGF-AB | Healthy | 13400 | 3400 | 91700 | 24100 | 6.8 |
| | OA | 16799 | 5731 | 37889 | 24922 | 2.5 |
| PDGF-BB | Healthy | 4702 | 1027 | 23810 | 6126 | 5.1 |
| | OA | 5306 | 2422 | 11936 | 8655 | 2.5 |
| IGF-I | Healthy | 114000 | 30000 | 155000 | 34000 | 1.4 |
| | OA | 79072 | 22137 | 118060 | 42827 | 1.5 |
| EGF | Healthy | 240 | 71 | 1227 | 300 | 5.1 |
| | OA | 374 | 199 | 707 | 489 | 2.2 |

(continued)

| Cytokine | | Baseline | | APS | | Fold Increase |
|---|---|---|---|---|---|---|
| | | Average | StDev | Average | StDev | Average |
| sTNF-RI | Healthy | 629 | 76 | 2408 | 338 | 3.8 |
| | OA | 808 | 275 | 3011 | 964 | 3.9 |
| TGF-β1 | Healthy | 25717 | 11131 | 181245 | 56420 | 7.1 |
| | OA | 56594 | 56940 | 153567 | 145973 | 4.2 |
| sIL-1RII | Healthy | 11,786 | ND | 26,000 | ND | 2.2 |
| | OA | ND | ND | ND | ND | ND |
| HGF | Healthy | 782 | ND | 3244 | ND | 4.1 |
| | OA | ND | ND | ND | ND | ND |

Example 5

Generation of a Protein Solution from Adipose Tissue

Adipose stromal cells are prepared as follows. Adipose tissue is minced into small pieces (about 1 cm3) and digested in 2 mg/mL type I collagenase (Worthington Biochemical Corp., Lakewood, N.J.) under intermittent mechanical agitation in a water bath at 37°C for 180 minutes. Digestion can be neutralized by the addition of medium or a blood-derived solution. The cell suspension is centrifuged (300×g for 7 minutes at 25°C) followed by removal of the supernatant from the cell pellet. The pellet is then re-suspended in a compatible solution to provide a liquid volume comprising adipose stromal cells.

Alternatively, the pellet is suspended with whole blood obtained from the subject, and added to a GPS™ Platelet Concentrate System, from Biomet Biologics, Inc. (Warsaw, Ind.). Following centrifugation, the platelet-rich plasma layer, which also contains the adipose stromal cells, is extracted from the system.

The adipose stromal cells, optionally including platelet-rich plasma, are then combined with polyacrylamide beads and subjected to a pulsed electromagnetic field by using a pair of Helmholtz coils to stimulate production of IL-1ra. The adipose stromal cells and polyacrylamide beads are separated from the liquid solution to obtain a solution rich in IL-1ra.

Example 6

Generation of Anti-inflammatory Protein Solution Composition From Lipoaspirate

A therapeutic composition of IL-1ra is generated from stromal cells isolated from adipose tissue. Isolation of human stromal cells is performed by obtaining human subcutaneous adipose tissue from lipoaspiration/liposuction procedures and digesting the tissue in collagenase type I solution (Worthington Biochemical Corp., Lakewood, N.J.) under gentle agitation for 1 hour at 37°C. The dissociated cells are filtered with 500 μm and 250 μm Nitex filters. The fraction is centrifuged at 300×g for 5 minutes. The supernatant is discarded and the cell pellet is re-suspended in a compatible liquid solution, such as a blood-derived solution.

Example 7

Determining Suitability of a Device by Using Efficacy Ratios

26 APS All-In-One kits, from Biomet Biologics, LLC (Warsaw, Ind., USA), were tested for their ability to generate an anti-inflammatory composition.

Whole blood was collected from 26 human donors. 10 mL of the whole blood was placed aside as input aliquots and 50 mL of whole was delivered to each device. The devices were centrifuged at 1750 x g for 15 min to generate an intermediate cell solution. The intermediate cell solution was then transferred into a desiccating bead chamber in which the cell solution was mixed with desiccating beads for 30 seconds. Then, the device was centrifuged at 1750 x g for 5 minutes. After centrifugation, an output anti-inflammatory composition was removed from each device.

The concentrations of IL-1β and IL-1ra were measured from the input aliquots and from the output anti-inflammatory compositions ELISA analysis. An Anti-IL-1 Efficacy Ratio was then determined for the input and output cytokine concentrations from each device by the following equation.

$$\text{Anti-IL-1 Efficacy Ratio} = \frac{\left(\frac{[\text{IL}-1\text{ra}]}{[\text{IL}-1\beta]}\right)\text{Output}}{\left(\frac{[[\text{IL}-1\text{ra}]}{[\text{IL}-1\beta]}\right)\text{Input}}$$

The Anti-IL-1 Efficacy Ratios are shown in Table 8.

Table 8. Anti-IL-1 Efficacy Ratios from 26 devices.

| Device # | 1 | 2 | 3 | 4 | 5 | 6 | 7 | 8 | 9 | 10 | 11 | 12 | 13 | |
|---|---|---|---|---|---|---|---|---|---|---|---|---|---|---|
| Ratio | 2.4 | 1.9 | 1.7 | 1.8 | 5.1 | 2.5 | 1.8 | 1.8 | 1.8 | 2.3 | 2.8 | 2.2 | 7.1 | |
| Device # | 14 | 15 | 16 | 17 | 18 | 19 | 20 | 21 | 22 | 23 | 24 | 25 | 26 | Average |
| Ratio | 2.6 | 4.3 | 2.2 | 1.9 | 1.4 | 2.5 | 3.5 | 1.4 | 1.2 | 2.0 | 1.7 | 1.2 | 3.5 | 2.5±1.3 |

The results in Table 8 show that each Plasmax™ device generated an anti-inflammatory composition with an Anti-IL-1 Efficacy Ratio of greater than 1. Therefore, each device increased the level of IL-1ra more than the level of IL-1β. Accordingly, the device is suitable for generating anti-inflammatory compositions for treating disorders associated with increased levels of IL-1β. Additionally, the anti-inflammatory compositions are suitable for administering to a patient with an inflammatory disorder associated with increased levels of IL-1β.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described.

Non-limiting Discussion of Terminology

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "prefer" or "preferable" refer to embodiments of the technology that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein. Further, as used herein the term "consisting essentially of' recited materials or components envisions embodiments "consisting of' the recited materials or components.

A" and "an" as used herein indicate "at least one" of the item is present; a plurality of such items may be present, when possible. "About" when applied to values indicates that the calculation or the measurement allows some slight imprecision in the value (with some approach to exactness in the value; approximately or reasonably close to the value; nearly). If, for some reason, the imprecision provided by "about" is not otherwise understood in the art with this ordinary meaning, then "about" as used herein indicates at least variations that may arise from ordinary methods of measuring or using such parameters.

As referred to herein, ranges are, unless specified otherwise, inclusive of endpoints and include disclosure of all distinct values and further divided ranges within the entire range. Thus, for example, a range of "from A to B" or "from about A to about B" is inclusive of A and of B. Disclosure of values and ranges of values for specific parameters (such as temperatures, molecular weights, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1-10, or 2-9, or 3-8, it is also envisioned that Parameter X may have other ranges of values including 1-9, 1-8, 1-3, 1-2, 2-10, 2-8, 2-3, 3-10, and 3-9.

**Claims**

1. A method for generating an anti-inflammatory composition for administration to a human or other animal subject, the method comprising:

(a) determining the input concentration of a first anti-inflammatory cytokine ([first anti-inflammatory cytokine] input), and the input concentration of a first inflammatory cytokine ([first inflammatory cytokine] input), in a biological material comprising the first inflammatory cytokine and the first anti-inflammatory cytokine which has been obtained from a human or other animal subject;
(b) processing the biological material to produce an anti-inflammatory composition having an increased concentration of the first anti-inflammatory cytokine;
(c) determining output concentration of the first anti-inflammatory cytokine ([first anti-inflammatory cytokine] output) and the output concentration of the first inflammatory cytokine ([first inflammatory cytokine] output), in the anti-inflammatory composition;
(d) calculating a first Efficacy Ratio pursuant to the formula:

$$\text{first Efficacy Ratio} = \frac{\left(\dfrac{[\text{first anti}-\text{inflammatory cytokine}]}{[\text{first inflammatory cytokine}]}\right)\text{Output}}{\left(\dfrac{[\text{first anti}-\text{inflammatory cytokine}]}{[\text{first inflammatory cytokine}]}\right)\text{Input}}$$

and;
(e) preparing the anti-inflammatory composition for administration to the subject if the first Efficacy Ratio is 1 or greater.

2. A method for assessing the efficacy of an anti-inflammatory composition, the method comprising:

(a) determining the input concentration of a first anti-inflammatory cytokine ([first anti-inflammatory cytokine] input), and the input concentration of a first inflammatory cytokine ([first inflammatory cytokine] input), in a biological material comprising the first inflammatory cytokine and the first anti-inflammatory cytokine which has been obtained from a human or other animal subject;

(b) processing the biological material to produce an anti-inflammatory composition having an increased concentration of the first anti-inflammatory cytokine;

(c) determining output concentration of the first anti-inflammatory cytokine ([first anti-inflammatory cytokine] output) and the output concentration of the first inflammatory cytokine ([first inflammatory cytokine] output), in the anti-inflammatory composition;

(d) calculating a first Efficacy Ratio pursuant to the formula:

$$\text{first Efficacy Ratio} = \frac{\left(\dfrac{[\text{first anti}-\text{inflammatory cytokine}]}{[\text{first inflammatory cytokine}]}\right)\text{Output}}{\left(\dfrac{[\text{first anti}-\text{inflammatory cytokine}]}{[\text{first inflammatory cytokine}]}\right)\text{Input}}$$

3. The method according to Claim 1 or 2, wherein the processing comprises contacting the biological material with a solid extraction material, the solid extraction material preferably comprising polyacrylamide.

4. The method according to any of Claims 1-3, wherein the biological material is selected from the group consisting of whole blood, a blood fraction such as white blood cells, adipose tissue, adipocytes, bone marrow aspirate, concentrated bone marrow aspirate, synovial fluid, urine, and mixtures thereof, optionally wherein the biological material is platelet rich plasma.

5. The method according to any of the preceding claims, wherein the first inflammatory cytokine comprises a plurality of inflammatory cytokines, and the first anti-inflammatory cytokine comprises a plurality of anti-inflammatory cytokines.

6. The method according to any of the preceding claims, wherein the anti-inflammatory cytokine comprises a cytokine selected from the group consisting of interleukin-1 receptor antagonist (IL-ira), soluble IL-1 receptor II (sIL-1RII), interleukin-4 (IL-4), interleukin-10 (IL-10), interleukin-13 (IL-13), soluble tumor necrosis factor receptor I (sTNF-RI), soluble tumor necrosis factor receptor II (sTNF-RII), and mixtures thereof.

7. The method according to claim 6, wherein

(a) the anti-inflammatory cytokine is selected from the group consisting of interleukin-1 receptor antagonist (IL-ira), soluble interleukin-1 receptor II (sIL-1RII), and combinations thereof, and the inflammatory cytokine is interleukin-1 (IL-1); or

(b) the anti-inflammatory cytokine is soluble tumor necrosis factor receptor I (sTNF-RI), or soluble tumor necrosis factor receptor II (sTNF-RII), and the inflammatory cytokine is tumor necrosis factor $\alpha$ (TNF$\alpha$).

8. The method according to any of the preceding claims, wherein the biological material further comprises a second inflammatory cytokine and a second anti-inflammatory cytokine, and the method further comprises

i. determining the input concentration of the second anti-inflammatory cytokine ([second anti-inflammatory cytokine] input), and the input concentration of the second inflammatory cytokine ([second inflammatory cytokine] input), in the biological material;

ii. determining the output concentration of the second anti-inflammatory cytokine ([second anti-inflammatory cytokine] output) and the output concentration of the second inflammatory cytokine ([second inflammatory cytokine] output), in the anti-inflammatory composition; and

iii. calculating a second Efficacy Ratio pursuant to the formula:

$$\text{second Efficacy Ratio} = \frac{\left(\dfrac{[\text{second anti} - \text{inflammatory cytokine}]}{[\text{second inflammatory cytokine}]}\right) \text{Output}}{\left(\dfrac{[\text{second anti} - \text{inflammatory cytokine}]}{[\text{second inflammatory cytokine}]}\right) \text{Input}}$$

and;

step (e) comprises transferring the anti-inflammatory composition into a delivery device or into a storage container if both the first Efficacy Ratio and the second Efficacy Ratio are 1 or greater.

9. The method according to Claim 8, wherein the first inflammatory cytokine and the second inflammatory cytokine are the same, and optionally:

(a) the first anti-inflammatory cytokine is interleukin-1 receptor antagonist (IL-ira), the second anti-inflammatory cytokine is soluble interleukin-1 receptor II (sIL-1RII), and the first inflammatory cytokine and the second inflammatory cytokine are both interleukin-1 (IL-1); or,
(b) the first anti-inflammatory cytokine is soluble tumor necrosis factor receptor I (sTNF-RI), the second anti-inflammatory cytokine is soluble tumor necrosis factor receptor II (sTNF-RII), and the first inflammatory cytokine and the second inflammatory cytokine are both tumor necrosis factor $\alpha$ (TNF$\alpha$).

10. The method according to Claim 8, wherein the first inflammatory cytokine and the second inflammatory cytokine are different, and optionally:

(a) the first anti-inflammatory cytokine is selected from the group consisting of IL-1ra, sIL-1RII, and mixtures thereof, and the first inflammatory cytokine is IL-i; and
(b) the second anti-inflammatory cytokine is selected from the group consisting of sTNF-RI, sTNF-RII and mixtures thereof, and the second inflammatory cytokine is TNF$\alpha$.

11. The method according to Claim 1, wherein the method comprises the use of a biological material processing device, or a diagnostic device for calculating Efficacy Ratios.

12. The method according to Claim 1, wherein the determining the input concentration step (a) and the determining the output concentration step (c) are performed using a diagnostic selected from the group consisting of western blotting, enzyme-linked immunosorbent assays (ELISA), high performance liquid chromatography (HPLC), liquid chromatography-mass spectroscopy (LCMS), and combinations thereof.

13. The method according to Claim 2, wherein the determining the input concentration step (a) and the determining the output concentration step (c) are performed using a diagnostic selected from the group consisting of western blotting, enzyme-linked immunosorbent assays (ELISA), high performance liquid chromatography (HPLC), liquid chromatography-mass spectroscopy (LCMS), surface plasmon resonance (SPR) based techniques, radioimmunoassay (RIA) techniques, and combinations thereof.

14. The method according to Claim 1, wherein the preparing of the anti-inflammatory composition comprises storing the composition under conditions suitable to maintain the safety and efficacy of the composition.

15. The method according to Claim 1, wherein processing the biological material comprises contacting the biological material with a solid extraction material configured to concentrate the first anti-inflammatory cytokine.

**Patentansprüche**

1. Verfahren zum Erzeugen einer antiinflammatorischen Zusammensetzung zur Verabreichung an einen Menschen oder ein anderes tierisches Subjekt, wobei das Verfahren folgendes umfasst:

(a) Bestimmen der Eingangskonzentration eines ersten antiinflammatorischen Zytokins ([erstes antiinflammatorisches Zytokin] Eingang) und der Eingangskonzentration eines ersten inflammatorischen Zytokins ([erstes iinflammatorisches Zytokin] Eingang) in einem biologischen Material, welches das erste inflammatorische Zytokin und das erste antiinflammatorische Zytokin umfasst, das von einem Menschen oder einem anderen tierischen Subjekt erhalten worden ist;

(b) Verarbeiten des biologischen Materials, um eine antiinflammatorische Zusammensetzung zu erzeugen, die eine erhöhte Konzentration des ersten antiinflammatorischen Zytokins aufweist;

(c) Bestimmen der Ausgangskonzentration des ersten antiinflammatorischen Zytokins ([erstes antiinflammatorisches Zytokin] Ausgang) und der Ausgangskonzentration des ersten inflammatorischen Zytokins ([erstes iinflammatorisches Zytokin] Ausgang) in der antiinflammatorischen Zusammensetzung;

(d) Berechnen eines ersten Wirksamkeitsverhältnisses gemäß der Formel:

$$erstes\ Wirksamkeitsverh\ddot{a}ltnis = \frac{\left(\frac{[erstes\ antiinflammatorisches\ Zytokin]}{[erstes\ inflammatorisches\ Zytokin]}\right)Ausgang}{\left(\frac{[erstes\ antiinflammatorisches\ Zytokin]}{[erstes\ inflammatorisches\ Zytokin]}\right)Eingang};$$

und

(e) Vorbereiten der antiinflammatorischen Zusammensetzung für eine Verabreichung an das Subjekt, wenn das erste Wirksamkeitsverhältnis gleich 1 oder größer ist.

2.  Verfahren zur Beurteilung der Wirksamkeit einer antiinflammatorischen Zusammensetzung, wobei das Verfahren folgendes umfasst:

(a) Bestimmen der Eingangskonzentration eines ersten antiinflammatorischen Zytokins ([erstes antiinflammatorisches Zytokin] Eingang) und der Eingangskonzentration eines ersten inflammatorischen Zytokins ([erstes iinflammatorisches Zytokin] Eingang) in einem biologischen Material, welches das erste inflammatorische Zytokin und das erste antiinflammatorische Zytokin umfasst, das von einem Menschen oder einem anderen tierischen Subjekt erhalten worden ist;

(b) Verarbeiten des biologischen Materials, um eine antiinflammatorische Zusammensetzung zu erzeugen, die eine erhöhte Konzentration des ersten antiinflammatorischen Zytokins aufweist;

(c) Bestimmen der Ausgangskonzentration des ersten antiinflammatorischen Zytokins ([erstes antiinflammatorisches Zytokin] Ausgang) und der Ausgangskonzentration des ersten inflammatorischen Zytokins ([erstes iinflammatorisches Zytokin] Ausgang) in der antiinflammatorischen Zusammensetzung;

(d) Berechnen eines ersten Wirksamkeitsverhältnisses gemäß der Formel:

$$erstes\ Wirksamkeitsverh\ddot{a}ltnis = \frac{\left(\frac{[erstes\ antiinflammatorisches\ Zytokin]}{[erstes\ inflammatorisches\ Zytokin]}\right)Ausgang}{\left(\frac{[erstes\ antiinflammatorisches\ Zytokin]}{[erstes\ inflammatorisches\ Zytokin]}\right)Eingang}.$$

3.  Verfahren nach Anspruch 1 oder 2, wobei das Verarbeiten das Inkontaktbringen des biologischen Materials mit einem festen Extraktionsmaterial umfasst, wobei das feste Extraktionsmaterial vorzugsweise Polyacrylamid umfasst.

4.  Verfahren nach einem der Ansprüche 1 bis 3, wobei das biologische Material ausgewählt ist aus der Gruppe bestehend aus Vollblut, einem Blutanteil, wie etwa weiße Blutkörperchen, Fettgewebe, Adipozyten, Knochenmarkaspirat, konzentriertem Knochenmarkaspirat, Gelenkflüssigkeit, Urin und Mischungen davon, wobei das biologische Material optional plättchenreiches Plasma ist.

5.  Verfahren nach einem der vorstehenden Ansprüche, wobei das erste inflammatorische Zytokin eine Mehrzahl inflammatorischer Zytokine umfasst, und wobei das erste antiinflammatorische Zytokin eine Mehrzahl antiinflammatorischer Zytokine umfasst.

6.  Verfahren nach einem der vorstehenden Ansprüche, wobei das antiinflammatorische Zytokin ein Zytokin umfasst, das ausgewählt ist aus der Gruppe bestehend aus Interleukin-1-Rezeptorantagonist (IL-1RA), löslichem IL-1-Rezeptor-II (sIL-1RII), Interleukin-4 (IL-4), Interleukin-10 (IL-10), Interleukin-13 (IL-13), löslichem Tumornektrosefaktor-Rezeptor-I (sTNF-RI), löslichem Tumornekrose-Rezeptor-II (sTNF-RII) und Mischungen davon.

7.  Verfahren nach Anspruch 6, wobei:

(a) das antiinflammatorische Zytokin ausgewählt ist aus der Gruppe bestehend aus Interleukin-1-Rezeptorantagonist (IL-1RA), löslichem Interleukin-1-Rezeptor II (sIL-1 RII) und Kombinationen davon, und wobei das inflammatorische Zytokin Interleukin-1 (IL-1) ist; oder

(b) das antiinflammatorische Zytokin löslicher Tumornektrosefaktor-Rezeptor-I (sTNF-RI) oder löslicher Tumornektrosefaktor-Rezeptor-II (sTNF-RII) ist, und wobei das inflammatorische Zytokin Tumornekrosefaktor $\alpha$ (TNF$\alpha$) ist.

8. Verfahren nach einem der vorstehenden Ansprüche, wobei das biologische Material ferner ein zweites inflammatorisches Zytokin und ein zweites antiinflammatorisches Zytokin umfasst, und wobei das Verfahren ferner folgendes umfasst:

i) Bestimmen der Eingangskonzentration des zweiten antiinflammatorischen Zytokins ([zweites antiinflammatorisches Zytokin] Eingang) und der Eingangskonzentration des zweiten inflammatorischen Zytokins ([zweites iinflammatorisches Zytokin] Eingang) in dem biologischen Material;

ii) Bestimmen der Ausgangskonzentration des zweiten antiinflammatorischen Zytokins ([zweites antiinflammatorisches Zytokin] Ausgang) und der Ausgangskonzentration des zweiten inflammatorischen Zytokins ([zweites iinflammatorisches Zytokin] Ausgang) in der antiinflammatorischen Zusammensetzung; und

iii) Berechnen eines zweiten Wirksamkeitsverhältnisses gemäß der Formel:

$$zweites\ Wirksamkeitsverh\ddot{a}ltnis = \frac{\left(\frac{[zweites\ antiinflammatorisches\ Zytokin]}{[zweites\ inflammatorisches\ Zytokin]}\right)Ausgang}{\left(\frac{[zweites\ antiinflammatorisches\ Zytokin]}{[zweites\ inflammatorisches\ Zytokin]}\right)Eingang}\ ;$$

und

wobei Schritt (e) das Übertragen der antiinflammatorischen Zusammensetzung in eine Zufuhrvorrichtung oder in einen Lagerbehälter umfasst, wenn das erste Wirksamkeitsverhältnis und das zweite Wirksamkeitsverhältnis jeweils gleich 1 oder größer ist.

9. Verfahren nach Anspruch 8, wobei das erste inflammatorische Zytokin und das zweite inflammatorische Zytokin identisch sind, und wobei optional:

(a) das erste antiinflammatorische Zytokin Interleukin-1-Rezeptorantagonist (IL-1RA) ist, das zweite antiinflammatorische Zytokin löslicher Interleukin-1-Rezeptor-II (sIL-1RII) ist, und das erste inflammatorische Zytokin und das zweite inflammatorische Zytokin beide Interleukin-1 (IL-1) sind; oder

(b) das erste antiinflammatorische Zytokin löslicher Tumornekrosefaktor-Rezeptor-I (sTNF-RI) ist, das zweite antiinflammatorische Zytokin löslicher Tumornekrosefaktor-Rezeptor-II (sTNF-RII) ist, und das erste inflammatorische Zytokin und das zweite inflammatorische Zytokin beide Tumornekrosefaktor $\alpha$ (TNF$\alpha$) sind.

10. Verfahren nach Anspruch 8, wobei das erste inflammatorische Zytokin und das zweite inflammatorische Zytokin unterschiedlich sind, und wobei optional:

(a) das erste antiinflammatorische Zytokin ausgewählt ist aus der Gruppe bestehend aus IL-1RA, sIL-1 RII und Mischungen davon, und wobei das erste inflammatorische Zytokin IL-1 ist; und

(b) das zweite antiinflammatorische Zytokin ausgewählt ist aus der Gruppe bestehend aus sTNF-RI, sTNF-RII und Mischungen davon, und wobei das erste inflammatorische Zytokin TNF$\alpha$ ist.

11. Verfahren nach Anspruch 1. wobei das Verfahren die Verwendung einer Verarbeitungsvorrichtung für biologisches Material oder einer diagnostischen Vorrichtung zur Berechnung von Wirksamkeitsverhältnissen umfasst.

12. Verfahren nach Anspruch 1, wobei Schritt (a) des Bestimmens der Eingangskonzentration und Schritt (c) des Bestimmens der Ausgangskonzentration unter Verwendung einer Diagnostik ausgeführt werden, die ausgewählt ist aus der Gruppe bestehend aus Western Blot, ELISA (Enzyme-Linked Immunosorbent Assays), Hochleistungsflüssigkeitschromatographie (HPLC), Flüssigkeitschromatographie mit Massenspektrometrie-Kopplung (LCMS) und Kombinationen dieser.

13. Verfahren nach Anspruch 2, wobei Schritt (a) des Bestimmens der Eingangskonzentration und Schritt (c) des Bestimmens der Ausgangskonzentration unter Verwendung einer Diagnostik ausgeführt werden, die ausgewählt ist aus der Gruppe bestehend aus Western Blot, ELISA (Enzyme-Linked Immunosorbent Assays), Hochleistungsflüssigkeitschromatographie (HPLC), Flüssigkeitschromatographie mit Massenspektrometrie-Kopplung (LCMS), Techniken auf der Basis von Oberflächenplasmonresonanzspektroskopie (SPR), Radioimmunassay (RIA)-Techniken und Kombinationen dieser.

14. Verfahren nach Anspruch 1, wobei das Vorbereiten der antiinflammatorischen Zusammensetzung das Lagern der Zusammensetzung unter Bedingungen umfasst, die geeignet sind, um die Sicherheit und Wirksamkeit der Zusammensetzung aufrechtzuerhalten.

15. Verfahren nach Anspruch 1, wobei das Verarbeiten des biologischen Materials das Inkontaktbringen des biologischen Materials mit einem festen Extraktionsmaterial umfasst, das so gestaltet ist, dass es das erste antiinflammatorische Zytokin konzentriert.

**Revendications**

1. Procédé de génération d'une composition anti-inflammatoire destinée à l'administration chez un sujet humain ou chez un autre sujet animal, le procédé comprenant :

   (a) la détermination de la concentration d'entrée d'une première cytokine anti-inflammatoire ([première cytokine anti-inflammatoire] entrée), et de la concentration d'entrée d'une première cytokine inflammatoire ([première cytokine inflammatoire] entrée), dans un matériau biologique comprenant la première cytokine inflammatoire et la première cytokine anti-inflammatoire qui a été obtenue à partir d'un sujet humain ou d'un autre sujet animal ;
   (b) le traitement du matériau biologique afin de produire une composition anti-inflammatoire ayant une concentration accrue de la première cytokine anti-inflammatoire ;
   (c) la détermination d'une concentration de sortie de la première cytokine anti-inflammatoire ([première cytokine anti-inflammatoire] sortie) et de la concentration de sortie de la première cytokine inflammatoire ([première cytokine inflammatoire] sortie), dans la composition anti-inflammatoire ;
   (d) le calcul d'un premier rapport d'efficacité selon la formule :

$$\text{premier rapport d'efficacité} = \frac{\left(\frac{[\text{première cytokine anti} - \text{inflammatoire}]}{[\text{première cytokine inflammatoire}]}\right)\text{Sortie}}{\left(\frac{[\text{première cytokine anti} - \text{inflammatoire}]}{[\text{première cytokine inflammatoire}]}\right)\text{Entrée}}$$

   et ;
   (e) la préparation de la composition anti-inflammatoire destinée à une administration chez le sujet s'y le premier rapport d'efficacité est supérieur ou égal à 1.

2. Procédé d'évaluation de l'efficacité d'une composition anti-inflammatoire, le procédé comprenant :

   (a) la détermination la concentration d'entrée d'une première cytokine anti-inflammatoire ([première cytokine anti-inflammatoire] entrée), et de la concentration d'entrée d'une première cytokine inflammatoire ([première cytokine inflammatoire] entrée), dans un matériau biologique comprenant la première cytokine inflammatoire et la première cytokine anti-inflammatoire qui a été obtenue à partir d'un sujet humain ou d'un autre sujet animal ;
   (b) le traitement du matériau biologique afin de produire une composition anti-inflammatoire ayant une concentration accrue de la première cytokine anti-inflammatoire ;
   (c) la détermination d'une concentration de sortie de la première cytokine anti-inflammatoire ([première cytokine anti-inflammatoire] sortie) et de la concentration de sortie de la première cytokine inflammatoire ([première cytokine inflammatoire] sortie), dans la composition anti-inflammatoire ;
   (d) le calcul d'un premier rapport d'efficacité selon la formule :

$$\text{premier rapport d'efficacité} = \frac{\left(\dfrac{[\text{première cytokine anti} - \text{inflammatoire}]}{[\text{première cytokine inflammatoire}]}\right)\text{Sortie}}{\left(\dfrac{[\text{première cytokine anti} - \text{inflammatoire}]}{[\text{première cytokine inflammatoire}]}\right)\text{Entrée}}$$

3. Procédé selon la revendication 1 ou 2, dans lequel le traitement comprend la mise en contact du matériau biologique avec un matériau d'extraction solide, le matériau d'extraction solide comprenant de préférence du polyacrylamide.

4. Procédé selon l'une quelconque des revendications 1 à 3, dans lequel le matériau biologique est choisi dans le groupe constitué du sang total, d'une fraction de sang telle que des globules blancs, du tissu adipeux, d'adipocytes, d'un produit d'aspiration de moelle osseuse, d'un produit d'aspiration de moelle osseuse concentrée, de fluide synovial, d'urine, et des mélanges de ceux-ci, dans lequel éventuellement le matériau biologique est du plasma riche en plaquettes.

5. Procédé l'une quelconque des revendications précédentes, dans lequel la première cytokine inflammatoire comprend une pluralité de cytokines inflammatoires, et la première cytokine anti-inflammatoire comprend une pluralité de cytokines anti-inflammatoires.

6. Procédé l'une quelconque des revendications précédentes, dans lequel la cytokine anti-inflammatoire comprend une cytokine choisi dans le groupe constitué d'un antagoniste du récepteur d'interleukine-1 (IL-1ra), du récepteur II soluble IL-1 (sIL-1RII), de l'interleukine-4 (IL-4), de l'interleukine-10 (IL-10), de l'interleukine-13 (IL-13), du récepteur I soluble du facteur de nécrose tumorale (sTNF-RI), du récepteur II soluble du facteur de nécrose tumorale (sTNF-RII), et des mélanges de ceux-ci.

7. Procédé selon la revendication 6, dans lequel

   (a) la cytokine anti-inflammatoire est choisie dans le groupe constitué d'un antagoniste du récepteur d'interleukine-1 (IL-1ra), du récepteur II soluble d'interleukine-1 (sIL-1RII), et de combinaisons de ceux-ci, et la cytokine inflammatoire est l'interleukine-1 (IL-1) ; ou
   (b) la cytokine anti-inflammatoire est le récepteur I soluble du facteur de nécrose tumorale (sTNF-RI), ou le récepteur II soluble du facteur de nécrose tumorale (sTNF-RII), et la cytokine inflammatoire est le facteur $\alpha$ de nécrose tumorale (TNF$\alpha$).

8. Procédé l'une quelconque des revendications précédentes, dans lequel le matériau biologique comprend en outre une seconde cytokine inflammatoire et une seconde cytokine anti-inflammatoire, et le procédé comprend en outre

   i. la détermination de la concentration d'entrée de la seconde cytokine anti-inflammatoire ([seconde cytokine anti-inflammatoire] entrée), et de la concentration d'entrée de la seconde cytokine inflammatoire ([seconde cytokine inflammatoire] entrée), dans le matériau biologique ;
   ii. la détermination de la concentration de sortie de la seconde cytokine anti-inflammatoire ([seconde cytokine anti-inflammatoire] sortie) et de la concentration de sortie de la seconde cytokine inflammatoire ([seconde cytokine inflammatoire] sortie), dans la composition anti-inflammatoire ; et
   iii. le calcul d'un second rapport d'efficacité selon la formule :

$$\text{second rapport d'efficacité} = \frac{\left(\dfrac{[\text{seconde cytokine anti} - \text{inflammatoire}]}{[\text{seconde cytokine inflammatoire}]}\right)\text{Sortie}}{\left(\dfrac{[\text{seconde cytokine anti} - \text{inflammatoire}]}{[\text{seconde cytokine inflammatoire}]}\right)\text{Entrée}}$$

   et;
   l'étape (e) comprend le transfert de la composition anti-inflammatoire dans un dispositif de distribution ou dans un récipient de stockage si le premier rapport d'efficacité et le second rapport d'efficacité sont tous les deux supérieurs ou égaux à 1.

9. Procédé selon la revendication 8, dans lequel la première cytokine inflammatoire et la seconde cytokine inflammatoire sont les mêmes, et éventuellement :

(a) la première cytokine anti-inflammatoire est un antagoniste du récepteur d'interleukine-1 (IL-1ra), la seconde cytokine anti-inflammatoire est un récepteur II soluble d'interleukine-1 (sIL-1RII), et la première cytokine inflammatoire et la seconde cytokine inflammatoire sont toutes les deux l'interleukine-1 (IL-1) ; ou,
(b) la première cytokine anti-inflammatoire est le récepteur I soluble du facteur de nécrose tumorale (sTNF-RI), la seconde cytokine anti-inflammatoire est le récepteur II soluble du facteur de nécrose tumorale (sTNF-RII), et la première cytokine inflammatoire et la seconde cytokine inflammatoire sont toutes les deux le facteur $\alpha$ de nécrose tumorale (TNF$\alpha$).

10. Procédé selon la revendication 8, dans lequel la première cytokine inflammatoire et la seconde cytokine inflammatoire sont différentes, et éventuellement :

(a) la première cytokine anti-inflammatoire est choisie dans le groupe constitué d'IL-1ra, de sIL-1RII, et des mélanges de ceux-ci, et la première cytokine inflammatoire est l'IL-1 ; et
(b) la seconde cytokine anti-inflammatoire est choisie dans le groupe constitué de sTNF-RI, de sTNF-RII et des mélanges de ceux-ci, et la seconde cytokine inflammatoire est le TNF$\alpha$.

11. Procédé selon la revendication 1, le procédé comprenant l'utilisation d'un dispositif de traitement de matériau biologique, ou d'un dispositif de diagnostique permettant de calculer des rapports d'efficacité.

12. Procédé selon la revendication 1, dans lequel l'étape (a) de la détermination de la concentration d'entrée et l'étape (c) de la détermination de la concentration de sortie sont réalisées au moyen d'un diagnostic choisi dans le groupe constitué des analyses western Blot, d'essais d'immuno-absorption enzymatique (ELISA), de la chromatographie liquide à haute performance (HPLC), de la chromatographie liquide couplée à la spectroscopie de masse (LCMS), et de combinaisons de ceux-ci.

13. Procédé selon la revendication 2, dans lequel l'étape (a) de la détermination de la concentration d'entrée et l'étape (c) de la détermination de la concentration de sortie sont réalisées au moyen d'un diagnostic choisi dans le groupe constitué des analyses western Blot, d'essais d'immuno-absorption enzymatique (ELISA), de la chromatographie liquide à haute performance (HPLC), de la chromatographie liquide couplée à la spectroscopie de masse (LCMS), de techniques basées sur la résonance plasmonique de surface (SPR), de techniques radio-immunologiques (RIA), et de combinaisons de ceux-ci.

14. Procédé selon la revendication 1, dans lequel la préparation de la composition anti-inflammatoire comprend le stockage de la composition dans des conditions adaptées pour maintenir la sûreté et l'efficacité de la composition.

15. Procédé selon la revendication 1, dans lequel le traitement du matériau biologique comprend la mise en contact du matériau biologique avec un matériau d'extraction solide conçu pour concentrer la première cytokine anti-inflammatoire.

Fig. 1

Fig. 2

**Fig. 3A**

**Fig. 3B**

Fig. 4

Fig. 5

600

OBTAIN A BIOLOGICAL MATERIAL
605

↓

DETERMINE INPUT CYTOKINE CONCENTRATIONS
610

↓

PROCESS THE BIOLOGICAL MATERIAL TO
FORM AN ANTI-INFLAMMATORY
COMPOSITION
615

↓

DETERMINE OUTPUT CYTOKINE
CONCENTRATIONS
620

↓

CALCULATE AN EFFICACY RATIO
625

↓

KEEP OR DISCARD THE ANTI-
INFLAMMATORY COMPOSITION
630

Fig. 6

700

IDENTIFY AN INFLAMMATORY
CYTOKINE WITH AN ELEVATED LEVEL

— 705

OBTAIN A BIOLOGICAL MATERIAL

— 710

COLLECT AN ALIQUOT OF THE BIOLOGICAL
MATERIAL

— 715

PROCESS THE BIOLOGICAL MATERIAL TO
FORM AN ANTI-INFLAMMATORY
COMPOSITION

— 720

COLLECT AN ALIQUOT OF THE ANTI-INFLAMMATORY
COMPOSITION

— 725

DETERMINE AN INPUT CONCENTRATION OF THE INFLAMMATORY
AND ANTI-INFLAMMATORY CYTOKINES IN THE BIOLOGICAL
MATERIAL AND AN OUTPUT CONCENTRATION OF THE
INFLAMMATORY AND ANTI-INFLAMMATORY CYTOKINES IN THE
OUTPUT ANTI-INFLAMMATORY COMPOSITION

— 730

CALCULATE AN EFFICACY RATIO

— 735

ADMINISTER THE ANTI-INFLAMMATORY
COMPOSITION IF THE EFFICACY RATIO IS AT
LEAST 1.0

— 740

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6599873 B, Sommer
- US 5075222 A, Hannum
- US 20050197293 A, Mellis
- US 6623472 B, Reincke
- US 6713246 B, Reinecke
- US 6759188 B, Reinecke
- WO 2012030593 A
- US 7992725 B, Leach
- US 5585007 A, Antanavich
- US 6398972 B, Blasetti
- US 6649072 B, Brandt
- US 6790371 B, Dolocek
- US 7011852 B, Sukavaneshvar
- US 7179391 B, Leach
- US 7374678 B, Leach
- US 7223346 B, Dorian
- US 7708152 B, Dorian
- US 7806276 B, Leach
- US 8048297 B, Leach

- US 20060278588 A, Woodell-May
- US 20090220482 A, Higgins
- US 20100055087 A, Higgins
- US 20110052561 A, Hoeppner
- US 20120172836 A, Higgins
- US 840562 A, Binder
- US 841083 A, Landrigan
- US 837480 A, O'Shaughnessey
- US 839280 A, Leach
- US 840129 A, Matusuka
- US 841103 A, Landrigan
- US 7553413 B, Dorian
- US 7694828 B, Swift
- US 20130259951 A, O'Connell
- US 7845499 B, Higgins
- US 6337072 B, Ford
- US 20010053764 A, Sims

### Non-patent literature cited in the description

- **AREND WP ; MALYAK M ; GUTHRIDGE CJ ; GABAY C.** Interleukin-1 receptor antagonist: role in biology. *Annu. Rev. Immunol.,* 1998, vol. 16, 27-55
- **WOODELL-MAY et al.** *Journal of Orthopaedic Research,* 01 September 2011, vol. 29 (9), 1320-26

- **WOODELL-MAY JE ; RIDDERMAN DN ; SWIFT MJ ; HIGGINS J.** Producing Accurate Platelet Counts for Platelet Rich Plasma: Validation of a Hematology Analyzer and Preparation Techniques for Counting. *J. Craniofac. Surg.,* September 2005, vol. 16 (5), 749-56